# EUROPEAN PATENT SPECIFICATION

(11) **EP 4 269 612 B1**
(45) Date of publication and mention of the grant of the patent: **25.06.2025**
(21) Application number: 21911036.8
(22) Date of filing: 24.12.2021
(51) Int. Cl.: C12Q 1/6853, C12N 15/113, C12Q 1/6876

(54) **NUCLEIC ACID AMPLIFICATION METHOD, PRIMER SET, PROBE, AND KIT FOR NUCLEIC ACID AMPLIFICATION METHOD**
NUKLEINSÄUREAMPLIFIKATIONSVERFAHREN, PRIMERSATZ, SONDE UND KIT FÜR NUKLEINSÄUREAMPLIFIKATIONSVERFAHREN
PROCÉDÉ D'AMPLIFICATION D'ACIDE NUCLÉIQUE, ENSEMBLE D'AMORCES, SONDE ET KIT POUR PROCÉDÉ D'AMPLIFICATION D'ACIDE NUCLÉIQUE

(30) Priority: 24.12.2020 JP 2020215698
(43) Date of publication of application: 01.11.2023
(73) Proprietor: EIKEN KAGAKU KABUSHIKI KAISHA, Tokyo 110-8408 (JP)
(72) Inventor: ASANO Yoshinori, Ohtawara-shi, Tochigi 324-0036 (JP); MORI Yuta, Ohtawara-shi, Tochigi 324-0036 (JP); NAKAZATO Hiroaki, Shimotsuga-gun, Tochigi 329-0114 (JP); NOTOMI Tsugunori, Tokyo 110-8408 (JP)
(74) Representative: J A Kemp LLP
(86) International application number: PCT/JP2021/048115
(87) International publication number: WO 2022/138899

(56) References cited:
- EP-B1- 2 449 104
- WO-A1-2018/044831
- CN-A- 111 961 730
- JP-A- 2018 153 157
- US-A1- 2005 266 418
- ABDULLAH AL-MASKRI ABDU AHMED; YE JIAWEI; TALAP JADERA; HU HAIHONG; SUN LIANLI; YU LUSHAN; CAI SHENG; ZENG SU: "Reverse transcription-based loop-mediated isothermal amplification strategy for real-time miRNA detection with phosphorothioated probes", ANALYTICA CHIMICA ACTA, ELSEVIER, AMSTERDAM, NL, vol. 1126, 17 June 2020 (2020-06-17), AMSTERDAM, NL , pages 1 - 6, XP086231032, ISSN: 0003-2670, DOI: 10.1016/j.aca.2020.06.007

## Description

### [Technical Field]

The present invention relates to a method for amplifying nucleic acids, use of a primer and adaptor set in the nucleic acid amplification method, and use of a kit in the nucleic acid amplification method.

### [Background Art]

Conventionally, the microarray method, which captures and detects nucleic acids with probes immobilized on microarrays, is mainly used to detect short-chain nucleic acids such as fragmented DNA and miRNA. However, such a microarray method has problems that the operation is complicated and the equipment used is expensive.

Meanwhile, from the viewpoints of convenience and economy, the use of nucleic acid amplification methods such as PCR for detection of short-chain nucleic acids is also being studied. As such a nucleic acid amplification method, for example, US Patent Application Publication No. 2009/0220969 (PTL 1) describes a method including adding poly-A to miRNA and then amplifying it by PCR (Poly-A Tailing). Further, for example, US Patent Application Publication No. 2005/0266418 (PTL 2) describes a method including hybridizing a linker probe to miRNA to add a stem-loop structure and then amplifying it by PCR.

Furthermore, for example, Japanese Unexamined Patent Application Publication No. 2018-153157 (PTL 3) describes, as a method for amplifying a target RNA, a method including reverse transcribing the target RNA followed by extension and then amplifying it, and cites the LAMP method as an example of the amplification method. In the method described in PTL 3, based on a LAMP primer set that is a combination of a BIP primer comprising the B2 sequence and the B1c sequence, a FIP primer comprising the F2 sequence and the F1c sequence, a B3 primer comprising the B3 sequence, and an F3 primer comprising the F3 sequence, the reverse transcription primer and extension primer designed for the LAMP method have a structure in which the reverse transcription primer comprises the B1 sequence, B2 sequence, and B3 sequence in this order, and the extension primer comprises the F1 sequence, F2 sequence, and F3 sequence in this order.

In addition, for example, A. A. Abdullah AL-maskri et al., Analytica Chimica Acta 1126, 2020, 1-6 (NPL 1) describes a method in which a reverse transcription primer (SL probe) and extension primer (PS-H probe) having a stem-loop structure are used to reverse transcribe and extend the target RNA and then amplify it by the LAMP method. In the method described in NPL 1, the reverse transcription primer comprises the F1c sequence, F2 sequence, and F1 sequence in that order, and the extension primer comprises the B1c sequence, B2 sequence, and B1 sequence in this order, and both form a stem-loop structure. Also, in the method described in NPL 1, the B1c sequence and B2 sequence of the extension primer are phosphorothioated. After extension, the phosphorothioated B1c sequence and B2 sequence undergo double strand dissociation and selffolding to form stem-loop structures at both ends, so that no outer primer is required for the LAMP method. PTL 4 discloses chimeric primers with hairpin conformations and methods of using the same primers for amplifying a target nucleic acid.

### [Citation List]

### [Patent Literature]

[PTL 1] US Patent Application Publication No. 2009/0220969
[PTL 2] US Patent Application Publication No. 2005/0266418
[PTL 3] Japanese Unexamined Patent Application Publication No. 2018-153157
[PTL 4] European Patent Publication EP 2449104 B1 [Non Patent Literature]
[NPL 1] A. A. Abdullah AL-maskri et al., Analytica Chimica Acta 1126, 2020, 1-6

### [Summary of Invention]

### [Technical Problem]

However, the methods for amplifying nucleic acids by PCR, as described in PTLs 1 and 2, have the problem of insufficient sensitivity and specificity, especially when detecting short-chain nucleic acids using blood or body fluids as specimens. Further, in the method as described in PTL 3, the target RNA is reverse transcribed and extended, resulting in a linear structure. The present inventors have found that such a structure has a problem that the detection sensitivity and specificity of amplified products are not sufficient in nucleic acid amplification using the LAMP method. Meanwhile, the method described in NPL 1, in which the target RNA is reverse transcribed and extended to have a stem-loop structure, improves sensitivity, but has problems of a large number of steps and inferior simplicity.

The present invention has been made in view of the above-mentioned problems of the related art, and aims to provide a nucleic acid amplification method capable of simply and specifically amplifying a nucleic acid using a short-chain nucleic acid such as fragmented DNA or miRNA as a template, as well as use of a primer and adapter set and a kit in the nucleic acid amplification method.

### [Solution to Problem]

The present inventors have made intensive research to achieve the above object, and have found that if a Bw adapter primer having a stem-loop structure is used to synthesize a complementary strand of the target region of the target nucleic acid by reverse transcription reaction or extension reaction, and then an Fw adapter nucleotide having a stem-loop structure and an extension-inhibiting modification at the 3'-end is used to further synthesize its complementary strand, it is possible to simply and specifically form a dumbbell structure in which stem-loop structures are added to both ends of the complementary strand of the target region. The present inventors have also found that if this is used as a template and the LAMP primer is allowed to act to perform the LAMP method, non-specific amplification is sufficiently suppressed even if the target nucleic acid is a short-chain nucleic acid such as fragmented DNA or miRNA, making it possible to specifically amplify the nucleic acid corresponding to the target region. The present inventors have further found that in addition to the simple and specific template synthesis as described above, the subsequent LAMP method does not require complicated temperature and cycle control, so that such a method makes it possible to more easily perform nucleic acid amplification than conventionally. Thus, the present invention has been completed.

The present invention provides:
[1] A method for amplifying nucleic acids, comprising:
   step A including annealing a Bw adapter primer comprising the following structure (a):

      5'-B1c-BL-B1-N3c-3' ... (a)

      [Here, N3c represents an annealing region composed of a base sequence complementary to a base sequence on a 3'-end side of a target region of a target nucleic acid, BL represents a loop region comprising base sequence B2, and B1c and B1 represent stem regions comprising mutually complementary base sequences and capable of forming a double strand.]
   and the target region, synthesizing a base sequence complementary to a base sequence on a 5'-end side of the target region starting from a 3'-end of the Bw adapter primer, and obtaining a first template nucleic acid comprising the following structure (b):

      5'-B1c-BL-B1-N3c-N5c-3' ... (b)

      [Here, N5c represents the base sequence complementary to the base sequence on the 5'-end side of the target region, and a base sequence comprising N3c and N5c represents base sequence Nc complementary to the target region.]
   in which a stem-loop structure is added to a 5'-end of a complementary strand of the target region;
   step B including annealing an Fw adapter nucleotide comprising the following structure (c):

      5'-F1c-FL-F1-N5'-3' ... (c)

      [Here, N5' represents an annealing region composed of a base sequence complementary to base sequence N5c of the first template nucleic acid, FL represents a loop region comprising base sequence F2, and F1c and F1 represent stem regions comprising mutually complementary base sequences and capable of forming a double strand.]
      and having an extension-inhibiting modification at a 3'-end
   and the first template nucleic acid, synthesizing a complementary strand of the Fw adapter nucleotide starting from a 3'-end of the first template nucleic acid, and obtaining a second template nucleic acid comprising the following structure (d):

      5'-B1c-BL-B1-N3c-N5c-F1c-FLc-F1-3' ... (d)

      [Here, FLc represents a base sequence complementary to FL of the Fw adapter nucleotide.]
   in which a stem-loop structure is added to a 3'-end of the first template nucleic acid; and
   step C including amplifying base sequence Nc of the second template nucleic acid by a LAMP method by using an Fw inner primer comprising the following structure (e):

      5'-F1c-F2-3' ... (e)
   and a Bw inner primer comprising the following structure (f):

      5'-B1c-B2-3' ... (f)
   with the second template nucleic acid as a template.
[2] The nucleic acid amplification method according to [1], wherein a length of the base sequence Nc is 10 to 100 bases long.
[3] The nucleic acid amplification method according to [1] or [2], wherein the target nucleic acid is miRNA.
[4] The nucleic acid amplification method according to any one of [1] to [3], wherein step A and step B proceed in the same reaction system.
[5] The nucleic acid amplification method according to any one of [1] to [4], further comprising: a heat treatment step of heating the reaction product of step B to 85°C or higher after step B and before step C.
[6] The nucleic acid amplification method according to any one of [1] to [5], further comprising: after step C, a detection step of detecting the base sequence Nc using a probe that hybridizes to at least part of the base sequence Nc or at least part of a complementary strand of the base sequence Nc.
[7] The nucleic acid amplification method according to [6], wherein in the probe, a length of a base sequence that hybridizes to the base sequence N3c is 5 bases long or less.
[8] The nucleic acid amplification method according to [6], wherein a full-length base sequence of the probe is not contained entirely in one of the base sequence N3c and the base sequence N5c.
[9] Use of a primer and adapter set in the nucleic acid amplification method according to any one of [1] to [8], wherein the primer and adapter set comprises:
   a Bw adapter primer comprising the following structure (a):

      5'-B1c-BL-B1-N3c-3' ... (a)

      [Here, N3c represents an annealing region composed of a base sequence complementary to a base sequence on a 3'-end side of a target region of a target nucleic acid, BL represents a loop region comprising base sequence B2, and B1c and B1 represent stem regions comprising mutually complementary base sequences and capable of forming a double strand.]
   and an Fw adapter nucleotide comprising the following structure (c):

      5'-F1c-FL-F1-N5'-3' ... (c)

      [Here, N5' represents an annealing region composed of a base sequence complementary to base sequence N5c of the first template nucleic acid, FL represents a loop region comprising base sequence F2, and F1c and F1 represent stem regions comprising mutually complementary base sequences and capable of forming a double strand.]
      and having an extension-inhibiting modification at the 3'-end.
[10] The use according to [9], wherein the primer and adapter set further comprises:
   an Fw inner primer comprising the following structure (e):

      5'-F1c-F2-3' ... (e)

      and
   a Bw inner primer comprising the following structure (f):

      5'-B1c-B2-3' ... (f).
[11] Use of a kit in the nucleic acid amplification method according to any one of [1] to [8], wherein the kit comprises:
   (i) the primer and adapter set according to [9] or [10]; and
   (ii) a probe that hybridizes to at least part of the base sequence Nc or at least part of a complementary strand of the base sequence Nc.

### [Claim 12]

The use according to [11], wherein in the probe, a length of a base sequence that hybridizes to the base sequence N3c is 5 bases long or less.

### [Claim 13]

The use according to [11], wherein a full-length base sequence of the probe is not contained entirely in one of the base sequence N3c and the base sequence N5c.

The reason why the configuration of the present invention achieves the above objects is not necessarily clear, but the present inventors speculate as follows. Specifically, in the present invention, first, a template nucleic acid for use in the LAMP method is synthesized based on the target nucleic acid. Here, a Bw adapter primer having a stem-loop structure is used to form a dimer with the target region of the target nucleic acid. Therefore, the stacking effect increases the thermal stability of the dimer (such as RNA/DNA heterozygous or DNA/DNA homozygous) and promotes complementary strand synthesis (reverse transcription reaction or extension reaction). In addition, after that, the Fw adapter nucleotide having a stem-loop structure is similarly used as a template to further promote complementary strand synthesis and change the structure of the template nucleic acid to a dumbbell structure with stem-loop structures added to both ends, so that the amplification reaction in the LAMP method proceeds efficiently. Furthermore, at this time, since the 3'-end of the Fw adapter nucleotide has an extension-inhibiting modification, non-specific amplification due to mismatch extension between the Bw adapter primer and the Fw adapter nucleotide is also sufficiently suppressed, exhibiting excellent specificity.

### [Advantageous Effects of Invention]

The present invention makes it possible to provide a nucleic acid amplification method capable of simply and specifically amplifying a nucleic acid using a short-chain nucleic acid such as fragmented DNA or miRNA as a template without limiting the primer design due to its full length, as well as use of a primer and adaptor set and use of a kit in the nucleic acid amplification method.

### [Brief Description of Drawings]

[Fig. 1] Fig. 1 is a schematic conceptual diagram showing an embodiment of the nucleic acid amplification method of the present invention.
[Fig. 2A] Fig. 2A shows the fluorescence intensityreaction time curves obtained using miR-21-5p in the verification of the heat treatment effect after the extension reaction in Test Example 2, where (a) shows the results without heat treatment, and (b) shows the results with heat treatment.
[Fig. 2B] Fig. 2B shows the fluorescence intensityreaction time curves obtained using Let-7a-5p in the verification of the heat treatment effect after the extension reaction in Test Example 2, where (a) shows the results without heat treatment, and (b) shows the results with heat treatment.
[Fig. 3A] Fig. 3A shows the fluorescence intensityreaction time curves obtained using miR-21-5p in the verification of the 3'-end phosphorylation effect of the Fw adapter nucleotide in Test Example 3, where (a) shows the results without phosphorylation and (b) shows the results with phosphorylation.
[Fig. 3B] Fig. 3B shows the fluorescence intensityreaction time curves obtained using Let-7a-5p in the verification of the 3'-end phosphorylation effect of the Fw adapter nucleotide in Test Example 3, where (a) shows the results without phosphorylation and (b) shows the results with phosphorylation.
[Fig. 4A] Fig. 4A shows melting curves obtained using miR-21-5p by fluorescence quenching probe detection in Test Example 4, where (a) shows the results with use of miR-21-5p region design probes, and (b) shows the results with use of loop region design probes.
[Fig. 4B] Fig. 4B shows melting curves obtained using Let-7a-5p by fluorescence quenching probe detection in Test Example 4, where (a) shows the results with use of Let-7a-5p region design probes, and (b) shows the results with use of loop region design probes.
[Fig. 5] Fig. 5 shows melting curves obtained in the verification of the probe design region of Test Example 5, showing the results with use of various fluorescence quenching probes, where (a) is with use of miRNA21Probe-1, (b) is with use of miRNA21Probe-2, (c) is with use of miRNA21Probe-3, (d) is with use of miRNA21Probe-4, (e) is with use of miRNA21Probe-5, (f) is with use of miRNA21Probe-6, (g) is with use of miRNA21Probe-7, and (h) is with use of miRNA21Probe-8.

### [Detailed Description]

### <Nucleic Acid Amplification Method>

The nucleic acid amplification method of the present invention is a method for amplifying nucleic acids, comprising:
step A including annealing a Bw adapter primer comprising the following structure (a):

   5'-B1c-BL-B1-N3c-3' ... (a)

   [Here, N3c represents an annealing region composed of a base sequence complementary to a base sequence on a 3'-end side of a target region of a target nucleic acid, BL represents a loop region comprising base sequence B2, and B1c and B1 represent stem regions comprising mutually complementary base sequences and capable of forming a double strand.]
and the target region, synthesizing a base sequence complementary to a base sequence on a 5'-end side of the target region starting from a 3'-end of the Bw adapter primer, and obtaining a first template nucleic acid comprising the following structure (b):

   5'-B1c-BL-B1-N3c-N5c-3' ... (b)

   [Here, N5c represents the base sequence complementary to the base sequence on the 5'-end side of the target region, and a base sequence comprising N3c and N5c represents base sequence Nc complementary to the target region.]
in which a stem-loop structure is added to a 5'-end of a complementary strand of the target region;
step B including annealing an Fw adapter nucleotide comprising the following structure (c):

   5'-F1c-FL-F1-N5'-3' ... (c)

   [Here, N5' represents an annealing region composed of a base sequence complementary to base sequence N5c of the first template nucleic acid, FL represents a loop region comprising base sequence F2, and F1c and F1 represent stem regions comprising mutually complementary base sequences and capable of forming a double strand.]
   and having an extension-inhibiting modification at a 3'-end
and the first template nucleic acid, synthesizing a complementary strand of the Fw adapter nucleotide starting from a 3'-end of the first template nucleic acid, and obtaining a second template nucleic acid comprising the following structure (d):

   5'-B1c-BL-B1-N3c-N5c-F1c-FLc-F1-3' ... (d)

   [Here, FLc represents a base sequence complementary to FL of the Fw adapter nucleotide.]
in which a stem-loop structure is added to a 3'-end of the first template nucleic acid; and
step C including amplifying base sequence Nc of the second template nucleic acid by a LAMP method by using an Fw inner primer comprising the following structure (e):

   5'-F1c-F2-3' ... (e)

   and a Bw inner primer comprising the following structure (f) :

   5'-B1c-B2-3' ... (f)
with the second template nucleic acid as a template.

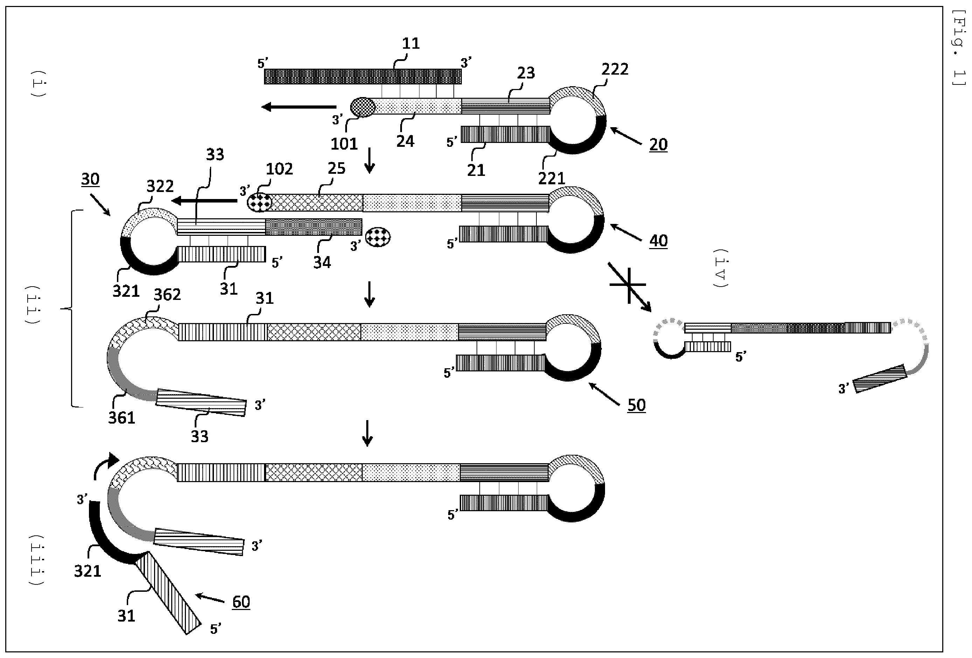

Hereinafter, preferred embodiments of the nucleic acid amplification method of the present invention will be described in detail, citing examples sometimes with reference to Fig. 1, but the present invention is not limited thereto. Fig. 1 is a schematic conceptual diagram showing an embodiment of the nucleic acid amplification method of the present invention. Note that in the following description and drawings, the same or corresponding elements are denoted by the same reference numerals, and overlapping descriptions are omitted.

### (Target Nucleic Acid)

In the present invention, the "target nucleic acid" is a nucleic acid that can contain the target region to be detected by the nucleic acid amplification method described later. In addition, in the present invention, a "nucleic acid" refers to a polynucleotide composed of deoxyribonucleotides and/or ribonucleotides, that is, DNA, RNA, or a polynucleotide of mixed deoxyribonucleotides and ribonucleotides, and may be fragments, modified in whole or in part, or may comprise non-natural nucleotides or specific sequences in whole or in part. Those modifications include, for example, methylation and deamination of DNA and/or RNA; labeling with radioisotopes and binding ligands (such as biotin and digoxin). Examples of the non-natural nucleotides include PNA (polyamide nucleic acid), LNA (registered trademark, locked nucleic acid), and ENA (registered trademark, 2'-O,4'-C-Ethylene-bridged nucleic acids). In addition, these nucleic acids are not particularly limited as long as the Bw adapter primer described later can be annealed to them, and may be single-stranded or double-stranded, and may have a three-dimensional structure such as a hairpin structure or a hammerhead structure. Among these, the target nucleic acid according to the present invention is preferably a short-chain nucleic acid such as a fragmented nucleic acid (such as fragmented DNA or fragmented RNA), miRNA, tRNA (transfer RNA), rRNA (ribosomal RNA), snRNA (small nuclear RNA), snoRNA (small nucleolar RNA), and siRNA (small interfering RNA), and more preferably miRNA, from the viewpoint that the nucleic acid amplification method of the present invention can be particularly effectively applied. Note that the "miRNA" usually refers to a single-stranded RNA molecule having a length of 20 to 25 bases, is not translated into protein, and is said to be mainly involved in post-transcriptional regulation of gene expression in eukaryotes.

Such a target nucleic acid is not particularly limited, and may be one extracted from a sample that may contain the target nucleic acid, or one that is artificially synthesized. The sample that may contain the target nucleic acid is not particularly limited. For example, depending on the purpose, it is possible to appropriately use solutions containing chemically synthesized nucleic acids, as well as various organisms (including cells, tissues, organs, and individuals) and extract fluids thereof; human and animal body fluids (such as saliva, tears, sweat, urine, blood, and lymph); plant biological fluids; biological culture fluids; environmental water (such as rivers, lakes, harbors, waterways, groundwater, purified water, sewage, and wastewater); suspensions of solids (such as soil and cinders). Moreover, the sample may be appropriately diluted with a diluent or suspended, or may be pH-adjusted. Examples of the diluent include buffers such as phosphate buffer, Tris buffer, Good's buffer, and borate buffer. As a method for extracting the target nucleic acid from the sample, a known method can be appropriately employed.

### (Target Region)

In the present invention, the "target region" refers to a region on the target nucleic acid that is intended to be detected by the nucleic acid amplification method of the present invention, and when the target nucleic acid is a short-chain nucleic acid such as a fragmented DNA or miRNA, the target region may be the entire target nucleic acid. In addition, in the following step A, it is preferably a single strand or a double strand in dynamic equilibrium. In order to show the correspondence with the following Bw adapter primer and Fw adapter nucleotide, in the following, the target region is referred to as the "target region N", the base sequence on the 5'-end side is referred to as the "base sequence N5", and the base sequence on the 3'-end side is referred to as the "base sequence N3" for convenience, as the case may be. Moreover, the target region N is composed of the base sequence N5 and the base sequence N3 (that is, N = 5'-N5-N3-3'), or contains an intervening base sequence between the base sequence N5 and the base sequence N3 (that is, N = 5'-N5-intervening base sequence-N3-3').

The length of the target region N according to the present invention is usually 5 to 5,000 bases long. In addition, the nucleic acid amplification method of the present invention can also be suitably performed for short-chain nucleic acids that are difficult to directly amplify, so that even if the target region N is 10 to 100 bases long, or even 20 to 50 bases long or 20 to 25 bases long, it can be used for the purpose of detection.

### (Bw Adapter Primer)

The "Bw adapter primer" according to the present invention is a primer comprising the following structure (a):

5'-B1c-BL-B1-N3c-3' ... (a)

.

In the structure (a), "N3c" represents an annealing region (24 in Fig. 1) composed of a base sequence complementary to a base sequence on a 3'-end side of the target region N of a target nucleic acid (11 in Fig. 1). Note that in the present specification, the base sequence on the target region N to which the annealing region N3c of the Bw adapter primer is annealed is referred to as the "base sequence N3". As a result, the Bw adapter primer (20 in Fig. 1) is annealed to the base sequence N3 of the target region N (in the present specification, "hybridize" is also used synonymously with anneal) to form a dimer.

In the present invention, a "base sequence complementary" to a certain sequence suffices to be a base sequence capable of hybridizing with each other to form a double strand, and does not have to be completely complementary. In the present invention, regarding the conditions for such hybridization, when "base sequence X and base sequence Y can form a double strand" or "base sequence X is a base sequence complementary to base sequence Y", the sequence complementarity between base sequence X and base sequence Y is preferably 80% or higher, 90% or higher, and 95% or higher (such as 96% or higher, 97% or higher, 98% or higher, and 99% or higher). Note that the sequence complementarity can be appropriately calculated by those skilled in the art using a known technique (such as BLAST (NCBI)).

In addition, in the present invention, regarding the conditions for such hybridization, when "base sequence X and base sequence Y can form a double strand" or "base sequence X is a base sequence complementary to base sequence Y", base sequence X may be a base sequence completely complementary to base sequence Y over the full length thereof, where 1 to 5 consecutive bases (preferably 1 to 3 bases, such as 3 bases, 2 bases, or 1 base) may be inserted, deleted or substituted at one or more sites.

To satisfy the above hybridization conditions, the base sequence constituting such annealing region N3c can be appropriately designed, according to the base sequences of the target region N of interest and the base sequence N3, more preferably so as not to hybridize to other regions.

The length of the annealing region N3c according to the present invention is preferably 5 to 200 bases long, more preferably 5 to 50 bases long.

In the structure (a), "BL" represents a loop region comprising the base sequence B2. The base sequence B2 is a sequence designed to anneal the Bw inner primer described later to the complementary strand of the second template nucleic acid (third template nucleic acid). Further, as a result, a loop structure composed of the loop region BL is added to the 5'-end side of the complementary strand of the target region N.

The loop region BL may be a region composed only of the base sequence B2 corresponding to the Bw inner primer described later, or may be a region containing a spacer sequence (spacer BS) on its 5'-end side and/or 3'-end side (preferably 3'-end side). Fig. 1, shows, as an example of the loop region BL, a configuration composed of the base sequence B2 (221 in Fig. 1) and the spacer BS on its 3'-end (222 in Fig. 1), but the configuration is not limited to this.

When the loop region BL contains a spacer BS, the length of the spacer BS (if present at both ends of the 5'-end side and the 3'-end side of the base sequence B2, the sum of the lengths thereof) is preferably 1 to 500 bases long, preferably 1 to 100 bases long, and more preferably 10 to 70 bases long.

In addition, the length of the base sequence B2 is preferably 5 to 200 bases long, more preferably 10 to 50 bases.

Furthermore, the length of the loop region BL corresponds to the length of the base sequence B2 when not containing the spacer BS, and corresponds to the sum of the length of the spacer BS and the length of the base sequence B2 when containing the spacer BS, and is more preferably 5 to 300 bases long, further preferably 20 to 120 bases long. When the length of the loop region BL is less than the lower limit, the stem-loop structure tends to fail to form or decrease in stability. Meanwhile, when the upper limit is exceeded, self-annealing is not preferentially performed, and mismatches with other primers tend to occur easily.

In the structure (a), "B1c" and "B1" represent stem regions comprising mutually complementary base sequences and capable of forming a double strand. The stem region B1c and stem region B1 are complementary base sequences designed to hybridize with each other to form a stem structure (double strand). Further, as a result, a stem-loop structure composed of the loop region BL, the stem region B1c, and the stem region B1 is added to the 5'-end side of the complementary strand of the target region N.

The stem region B1c and the stem region B1 suffice to contain base sequences capable of forming a double strand with each other, and may contain a base sequence that does not form a double strand (intervening base sequence) on the 5'-end side of the stem region B1c and/or the 3'-end side of the stem region B1. Note that the length of such intervening base sequences is each individually preferably 5 bases long or less, more preferably 3 bases long or less.

The lengths of the base sequences capable of forming a double strand between the stem region B1c and stem region B1 are each preferably 5 to 200 bases long, more preferably 10 to 50 bases long. When the length is less than the lower limit, the stem-loop structure tends to fail to form or decrease in stability. Meanwhile, when the upper limit is exceeded, self-annealing is not preferentially performed, and mismatches with other primers tend to occur easily.

Furthermore, the total length of the stem region B1c and the total length of the stem region B1 correspond to the length of the base sequence capable of forming the double strand when not containing the intervening base sequence, and correspond to the sum of the length of the intervening base sequence and the length of the base sequence capable of forming the double strand when containing the intervening base sequence, and are more preferably 5 to 200 bases long, further preferably 10 to 50 bases long.

The base sequence of such a Bw inner primer can be appropriately designed so as to satisfy the above hybridization conditions based on the base sequences of the target region N and the base sequence N3, so as to form the above stem-loop structure, or so as not to result in a situation where the base sequence B2 contained in the loop structure is a sequence that allows the Bw inner primer to hybridize to other regions.

Moreover, the method for obtaining the Bw inner primer is not particularly limited, and a conventionally known method or a method based thereon can be appropriately employed. For example, it can be chemically synthesized using a commercially available synthesizer and produced as a synthetic DNA. In addition, the Bw inner primer does not have to be composed only of natural nucleotides (deoxyribonucleotides and/or ribonucleotides), or may be partially or wholly composed of, for example, the above-described non-natural nucleotides, and is preferably composed only of natural or non-natural deoxyribonucleotides from the viewpoint of stability.

### (First Template Nucleic Acid)

The "first template nucleic acid" according to the present invention is a nucleic acid having the following structure (b):

5'-B1c-BL-B1-N3c-N5c-3' ... (b)

.

The first template nucleic acid (40 in Fig. 1) is a nucleic acid in which a stem-loop structure is added to the 5'-end of the complementary strand of the target region N, synthesized by annealing the Bw adapter primer and the target region N (base sequence N3), starting from the 3'-end of the Bw adapter primer. The first template nucleic acid may be composed of deoxyribonucleotides, ribonucleotides, or a mixture thereof, and is preferably composed only of deoxyribonucleotides from the viewpoint of stability in nucleic acid amplification reaction.

In the structure (b), "N5c" represents a base sequence complementary to the base sequence N5 on the 5'-end side of the target region N, and represents the base sequence to which the annealing region N5' of the Fw adapter nucleotide described later is annealed. A base sequence containing N3c and N5c represents a base sequence Nc complementary to the target region N. In Fig. 1, the base sequence N5c (25) and the base sequence N3c (24) are adjacent, but in the base sequence Nc, an intervening base sequence (that is, a base sequence complementary to the intervening base sequence between the base sequence N5 and the base sequence N3) may further be contained between base sequence N5c and base sequence N3c.

### (Fw Adapter Nucleotide)

The "Fw adapter nucleotide" according to the present invention is a nucleotide comprising the following structure (c):

5'-F1c-FL-F1-N5'-3' ... (c)

and having an extension-inhibiting modification at the 3'-end.

In the structure (c), "N5'" represents an annealing region (34 in Fig. 1) composed of a base sequence complementary to the base sequence N5c (25 in Fig. 1) on the 3'-end side of the first template nucleic acid (40 in Fig. 1). As a result, the Fw adapter nucleotide (30 in Fig. 1) is annealed to the base sequence N5c of the first template nucleic acid to form a dimer.

The annealing region N5' according to the present invention is, in principle, the same sequence as the base sequence N5 of the target region N, but for example, when the target region N is an RNA and the Fw adapter nucleotide is a DNA, the sequences may correspond to each other. Further, as long as the base sequence N5c and the annealing region N5' satisfy the above-described hybridization conditions, the base sequence N5 and the annealing region N5' do not have to be completely corresponding sequences.

To satisfy the above hybridization conditions, the base sequence constituting such annealing region N5' can be appropriately designed, according to the base sequences of the first template nucleic acid and the base sequence N5c (or the target region N of interest and the base sequence N5), more preferably so as not to hybridize to other regions.

The length of the annealing region N5' according to the present invention is preferably 5 to 200 bases long, more preferably 5 to 50 bases long.

In the structure (c), "FL" represents a loop region comprising the base sequence F2. The base sequence F2 is a sequence designed to anneal the Fw inner primer described later to the second template nucleic acid. By using this as a template, a loop structure composed of the base sequence FLc described later, which is complementary to the loop region FL, is added to the 3'-end side of the first template nucleic acid.

The loop region FL may be a region composed only of the base sequence F2 corresponding to the Fw inner primer described later, or may be a region containing a spacer sequence (spacer FS) on its 5'-end side and/or 3'-end side (preferably 3'-end side). Fig. 1, shows, as an example of the loop region FL, a configuration composed of the base sequence F2 (321 in Fig. 1) and the spacer FS on its 3'-end (322 in Fig. 1), but the configuration is not limited to this.

When the loop region FL contains a spacer FS, the length of the spacer FS (if present at both ends of the 5'-end side and the 3'-end side of the base sequence F2, the sum of the lengths thereof) is preferably 1 to 500 bases long, preferably 1 to 100 bases long, and more preferably 10 to 70 bases long.

In addition, the length of the base sequence F2 is preferably 5 to 200 bases long, more preferably 10 to 50 bases.

Furthermore, the length of the loop region FL corresponds to the length of the base sequence F2 when not containing the spacer FS, and corresponds to the sum of the length of the spacer FS and the length of the base sequence F2 when containing the spacer FS, and is more preferably 5 to 300 bases long, further preferably 20 to 120 bases long. When the length of the loop region FL is less than the lower limit, the stem-loop structure tends to fail to form or decrease in stability. Meanwhile, when the upper limit is exceeded, self-annealing is not preferentially performed, and mismatches with other primers tend to occur easily.

In the structure (c), "F1c" and "F1" represent stem regions comprising mutually complementary base sequences and capable of forming a double strand. The stem region F1c and stem region F1 are complementary base sequences designed to hybridize with each other to form a stem structure (double strand). By using these as a template, a stem loop structure composed of the base sequence FLc complementary to the loop region FL and the base sequences complementary to the stem region F1c and stem region F1 (stem region F1 and stem region F1c, respectively) is added to the 3'-end side of the first template nucleic acid.

The stem region F1c and the stem region F1 suffice to contain base sequences capable of forming a double strand with each other, and may contain a base sequence that does not form a double strand (intervening base sequence) on the 5'-end side of the stem region F1c and/or the 3'-end side of the stem region F1. Note that the length of such intervening base sequences is each individually preferably 5 bases long or less, more preferably 3 bases long or less.

The lengths of the base sequences capable of forming a double strand between the stem region F1c and stem region F1 are each preferably 5 to 200 bases long, more preferably 10 to 50 bases long. When the length is less than the lower limit, the stem-loop structure tends to fail to form or decrease in stability. Meanwhile, when the upper limit is exceeded, self-annealing is not preferentially performed, and mismatches with other primers tend to occur easily.

Furthermore, the total length of the stem region F1c and the total length of the stem region F1 correspond to the length of the base sequence capable of forming the double strand when not containing the intervening base sequence, and correspond to the sum of the length of the intervening base sequence and the length of the base sequence capable of forming the double strand when containing the intervening base sequence, and are more preferably 5 to 200 bases long, further preferably 10 to 50 bases long.

The base sequence of such an Fw adapter nucleotide can be appropriately designed so as to satisfy the above hybridization conditions based on the base sequence of the first template nucleic acid based on the base sequences of the target region N and the base sequence N5, so as to form the above stem-loop structure, or so as not to result in a situation where the base sequence F2 contained in the loop structure is a sequence that allows the Fw inner primer to hybridize to other regions.

The method for obtaining the Fw adapter nucleotide is not particularly limited, and a conventionally known method or a method based thereon can be appropriately employed, as with the method for obtaining the Bw adapter primer. In addition, the Fw adapter nucleotide does not have to be composed only of natural nucleotides, or may be partially or wholly composed of, for example, the above-described non-natural nucleotides, and is preferably composed only of natural or non-natural deoxyribonucleotides from the viewpoint of stability.

In the present invention, the Fw adapter nucleotide has an extension-inhibiting modification at the 3'-end. This is expected to suppress non-specific amplification due to mismatch extension between the Bw adapter primer and the Fw adapter nucleotide. The extension-inhibiting modification at the 3'-end of the Fw adapter nucleotide is not particularly limited as long as it can inhibit the extension reaction at the 3'-end of the nucleotide. For example, the OH group at the 3'-position of the ribose at the end of the polynucleotide can be modified and replaced with another substance such as a phosphate group to thereby inhibit (preferably terminate) extension reactions by the polymerase, but the inhibition is not limited to this.

### (Second Template Nucleic Acid)

The "second template nucleic acid" according to the present invention is a nucleic acid having the following structure (d):

5'-B1c-BL-B1-N3c-N5c-F1c-FLc-F1-3' ... (d)

The second template nucleic acid (50 in Fig. 1) is a nucleic acid in which a stem-loop structure is added to the 3'-end of the first template nucleic acid, synthesized by annealing the Fw adapter nucleotide and the first template nucleic acid, starting from the 3'-end of the first template nucleic acid. The second template nucleic acid may be composed of deoxyribonucleotides, ribonucleotides, or a mixture thereof, and is preferably composed only of deoxyribonucleotides from the viewpoint of stability in nucleic acid amplification reaction.

In the structure (d), "FLc" represents a base sequence complementary to FL of the Fw adapter nucleotide. Therefore, the base sequence FLc may be a region corresponding to the loop region FL and composed only of a base sequence complementary to the base sequence F2 (in the present specification, referred to as the "base sequence F2c" as the case may be), or may be a region containing a base sequence complementary to the spacer FS (in the present specification, referred to as the "base sequence FSc" as the case may be) on its 5'-end side and/or 3'-end side. As an example of the base sequence FLc, Fig. 1 shows, but without limitation, a configuration composed of a base sequence F2c (361 in Fig. 1) complementary to the base sequence F2 and a base sequence FSc (362 in Fig. 1) complementary to the spacer FS on the 5'-end side thereof. Preferred embodiments of the base sequence FLc, the base sequence F2c, and the base sequence FSc correspond to the preferred embodiments listed above for the loop region FL, the base sequence F2, and the spacer FS, respectively.

### (Fw Inner Primer)

The "Fw inner primer" according to the present invention is a primer (60 in Fig. 1) comprising the following structure (e):

5'-F1c-F2-3' ... (e)

.

In the structure (e), "F1c" and "F2" are synonymous with "F1c" and "F2" in the above Fw adapter nucleotide, respectively. As a result, the base sequence F2 of the Fw inner primer is annealed to the base sequence F2c (361 in Fig. 1) on the loop structure of the second template nucleic acid (50 in Fig. 1), making it possible to synthesize a complementary strand of the second template nucleic acid (third template nucleic acid), starting from the 3'-end of that Fw inner primer.

The base sequence of such an Fw inner primer can be appropriately designed so as to correspond to the Fw adapter nucleotide and satisfy the above-described hybridization conditions. In addition, the method for obtaining the Fw inner primer is not particularly limited, and a conventionally known method or a method based thereon can be appropriately employed, as with the method for obtaining the Bw adapter primer. The Fw inner primer does not have to be composed only of natural nucleotides, or may be partially or wholly composed of, for example, the above-described non-natural nucleotides, and is preferably composed only of natural or non-natural deoxyribonucleotides from the viewpoint of stability.

### (Third Template Nucleic Acid)

The "third template nucleic acid" according to the present invention is a nucleic acid having the following structure (g) (not shown):

5'-F1c-FL-F1-N5"-N3"-B1c-BLc-B1-3' ... (g).

The third template nucleic acid is a complementary strand of the second template nucleic acid, synthesized by annealing the second template nucleic acid and the Fw inner primer, starting from the 3'-end of that Fw inner primer.

In the structure (g), "N5''" and "N3''" represent base sequences complementary to the base sequences N5c and N3c of the second template nucleic acid, respectively, and the base sequence containing N5'' and N3" represents a base sequence N'' complementary to the base sequence Nc of the second template nucleic acid (in the present specification, referred to as the "complementary strand N" of the base sequence Nc" as the case may be). The base sequence N'' is, in principle, the same sequence as the target region N, but for example, when the target region N is an RNA and the third template nucleic acid is a DNA, the sequences may correspond to each other.

In the structure (g), "BLc" represents a base sequence complementary to BL of the second template nucleic acid. Therefore, the base sequence BLc may be a region corresponding to the base sequence of the loop region BL and composed only of a base sequence complementary to the base sequence B2 (in the present specification, referred to as the "base sequence B2c" as the case may be), or may be a region containing a base sequence complementary to the spacer BS (in the present specification, referred to as the "base sequence BSc" as the case may be) on its 5'-end side and/or 3'-end side. Preferred embodiments of the base sequence BLc, the base sequence B2c, and the base sequence BSc correspond to the preferred embodiments listed above for the loop region BL, the base sequence B2, and the spacer BS, respectively.

### (Bw Inner Primer)

The "Bw inner primer" according to the present invention is a primer comprising the following structure (f) (not shown):

5'-B1c-B2-3' ... (f).

In the structure (f), "B1c" and "B2" are synonymous with "B1c" and "B2" in the above Bw adapter primer, respectively. As a result, the base sequence B2 of the Bw inner primer is annealed to the base sequence B2c on the loop structure of the third template nucleic acid, making it possible to further synthesize a complementary strand of the third template nucleic acid, starting from the 3'-end of that Bw inner primer.

The base sequence of such an Bw inner primer can be appropriately designed so as to correspond to the Bw adapter primer and satisfy the above-described hybridization conditions. In addition, the method for obtaining the Bw inner primer is not particularly limited, and a conventionally known method or a method based thereon can be appropriately employed, as with the method for obtaining the Bw adapter primer. The Bw inner primer does not have to be composed only of natural nucleotides, or may be partially or wholly composed of, for example, the above-described non-natural nucleotides, and is preferably composed only of natural or non-natural deoxyribonucleotides from the viewpoint of stability.

In the Bw adapter primer and Fw adapter nucleotide, the annealing region N3c and annealing region N5' must each be individually designed according to the base sequence of the target region N, but other regions (such as the loop region BL and loop region FL; the stem regions B1c and B1 and stem regions F1c and F1) as well as the Fw inner primer and Bw inner primer do not necessarily have to be designed according to the base sequence of the target region N, and can have a common base sequence for detection of various target nucleic acids. Therefore, in the nucleic acid amplification method of the present invention, in designing individual primers and nucleotides, only the polynucleotide portions of the annealing region N3c and the annealing region N5' need to be designed according to the base sequence of the target region N, and the primers can be designed easily.

### (Step A)

The nucleic acid amplification method of the present invention first performs step A including annealing the Bw adapter primer and the target region N (base sequence N3), synthesizing a base sequence complementary to a base sequence on a 5'-end side of the target region N starting from a 3'-end of the Bw adapter primer, and obtaining the first template nucleic acid in which a stem-loop structure is added to a 5'-end of a complementary strand Nc of the target region N (in Fig. 1, (i) and upper part of (ii)).

In step A, when the Bw adapter primer and a complementary strand synthase are added to the target nucleic acid (or a sample that may comprise the target nucleic acid), for example, the Bw adapter primer is annealed to the target region N of interest, if present, and a base sequence complementary to the remaining base sequence on the 5'-end side of the target region N (base sequence N5c or intervening base sequence and base sequence N5c) is synthesized to obtain the first template nucleic acid.

Here, if the target nucleic acid is a double strand, it is preferably heated to make it single-stranded or in dynamic equilibrium. In this case, it is preferable to calculate Tm (melting temperature) based on the base sequence of the annealing region N3c of the Bw adapter primer according to the present invention, and heat at a temperature lower than the calculated Tm.

When the target nucleic acid is an RNA, the synthesis of a complementary strand of the target region N is preferably a reverse transcription reaction that synthesizes DNA from RNA. The complementary strand synthase for the reverse transcription reaction (that is, reverse transcriptase; 101 in Fig. 1) is not particularly limited, and usable examples thereof include, but are not limited to, one or a combination of two or more of Avian Myeloblastosis Virus (AMV)-derived recombinant enzymes (AMV), Cloned AMV, MML, V Recombinant HIV reverse transcriptase, Superscript II/III/IV, ReverTra Ace, Thermoscript, Omniscript, and Sensiscript. In this case, reaction conditions such as reaction temperature, time, and indicated pH can be appropriately adjusted according to the reverse transcriptase to be selected.

When the target nucleic acid is a DNA, the synthesis of the complementary strand of the target region N is preferably an extension reaction for extending DNA. The complementary strand synthetase (that is, DNA polymerase) in the case of the extension reaction is not particularly limited, and for example, it is possible to use one or a combination of two or more of Bst DNA polymerase, Bst 2.0 Warm Start DNA polymerase, Bca (exo-) DNA polymerase, Klenow fragment of E. coli DNA polymerase I, Vent DNA polymerase, Vent (Exo-) DNA polymerase (Vent DNA polymerase with exonuclease activity removed), Deep Vent DNA polymerase, Deep Vent (Exo-) DNA polymerase (Deep Vent DNA polymerase with exonuclease activity removed), Φ29 phage DNA polymerase, MS-2 phage DNA polymerase, and Csa DNA polymerase. Among these, Bst 2.0 Warm Start DNA polymerase is preferable from the viewpoint that non-specific amplification can be suppressed because of its relatively high activity and its Warm Start specifications. In this case, reaction conditions such as reaction temperature, time, and indicated pH can be appropriately adjusted according to the DNA polymerase to be selected.

### (Step B)

The nucleic acid amplification method of the present invention then performs step B including annealing the Fw adapter nucleotide and the first template nucleic acid, synthesizing a complementary strand of the Fw adapter nucleotide starting from a 3'-end of the first template nucleic acid, and obtaining the second template nucleic acid in which a stem-loop structure is added to a 3'-end of the first template nucleic acid (in Fig. 1, (ii)).

In step B, when the Fw adapter nucleotide and, if necessary, a complementary strand synthase are added to the reaction product of step A, for example, the Fw adapter nucleotide is annealed to the first template nucleic acid that is synthesized if the target region N of interest is present, and a base sequence complementary to the Fw adapter nucleotide: 5'-F1c-FLc-F1-3' is synthesized to obtain the second template nucleic acid.

The synthesis of the complementary strand of Fw adapter nucleotide is preferably an extension reaction for extending DNA. Examples of the complementary strand synthetase (that is, DNA polymerase) in the case of the extension reaction include those listed in step A above. In this case, reaction conditions such as reaction temperature, time, and indicated pH can be appropriately adjusted according to the DNA polymerase to be selected.

In addition, in the nucleic acid amplification method of the present invention, step A and step B can be performed in the same reaction system, which is preferable from the viewpoint of further convenience. When the target nucleic acid is an RNA and a DNA is used as each primer, for example, by using an isothermal strand displacement active polymerase enzyme as the complementary strand synthetase in step B, the reverse transcription reaction in step A and the extension reaction in step B can be performed simultaneously in the same container as the reverse transcriptase. Usable examples of the isothermal strand displacement active polymerase enzyme include, but are not limited to, one or a combination of two or more of Bst DNA polymerase, Bca (exo-) DNA polymerase, Klenow fragment of E. coli DNA polymerase I, Csa DNA polymerase, and Bst 2.0 Warm Start DNA polymerase. Among these, Bst 2.0 Warm Start DNA polymerase is preferable from the viewpoint that reverse transcription non-specific amplification can be suppressed because of its Warm Start specifications. In addition, examples of the reverse transcriptase in this case include Avian Myeloblastosis Virus (AMV)-derived recombinant enzyme (AMV).

When step A and step B are performed in the same reaction system, the reaction temperature is preferably 30 to 75°C, more preferably 40 to 70°C. Furthermore, the reaction time in this case is preferably 5 to 120 minutes, more preferably 15 to 60 minutes.

In step B, since the Fw adapter nucleotide has an extension-inhibiting modification at the 3'-end, the synthesis of the complementary strand of the first template nucleic acid starting from the 3'-end of the Fw adapter nucleotide is suppressed. Therefore, as a template nucleic acid, the synthesis of a nucleic acid having a structure similar to that of the third template nucleic acid as shown in (iv) of Fig. 1 is inhibited.

### (Heat Treatment Step)

The nucleic acid amplification method of the present invention preferably further includes a heat treatment step of heating the reaction product of step B to 85°C or higher before step C described later. This increases the efficiency of annealing of the Fw inner primer to the second template nucleic acid in the LAMP method described later, thereby improving sensitivity.

The temperature in the heat treatment is preferably 85 to 100°C, more preferably 90 to 100°C. Furthermore, the heat treatment time in this case is preferably 1 to 30 minutes, more preferably 1 to 10 minutes.

### (Step C)

The nucleic acid amplification method of the present invention then performs step C including amplifying base sequence Nc of the second template nucleic acid by a LAMP method by using the Fw inner primer and the Bw inner primer with the second template nucleic acid as a template (in Fig. 1, (iii)).

Since the second template nucleic acid has a so-called dumbbell-shaped structure having stem-loop structures at both ends as described above, it can be used as a template for amplification by the LAMP method to amplify the base sequence Nc. In other words, since the base sequence Nc is the complementary strand of the target region N, it becomes possible to indirectly amplify the target region N.

As an amplification method by the LAMP method, a conventionally known method or a method based thereon can be appropriately employed, and for example, the method described in International Publication No. 00/28082 can be employed.

More specifically, for example, the second template nucleic acid (or the reaction product of step B or the heat treatment step), the Fw inner primer, the Bw inner primer, and the complementary strand synthetase are mixed, and the temperature is maintained within a certain range, and in the presence of the second template nucleic acid, the following step proceeds.

### (Step 1)

First, in the second template nucleic acid, a DNA strand is synthesized by an extension reaction starting from the 3'-end (stem region F1) and using the self as a template. Here, the stem-loop structure on the 5'-end side is stripped to form a single strand. Furthermore, since the second template nucleic acid has a single-stranded loop structure (structure composed of the base sequence FLc) on the 3'-end side, the base sequence F2 of the Fw inner primer can be annealed thereto, whereby a DNA strand (third template nucleic acid) is synthesized using the second template nucleic acid as a template by an extension reaction starting from the 3'-end of the Fw inner primer. Here, the extension reaction of the third template nucleic acid proceeds while stripping the existing double strand.

### (Step 2)

On the other hand, the DNA strand, stripped to a single strand by the extension reaction of the third template nucleic acid starting from the Fw inner primer, has complementary base sequences, that is, B1c and B1, on the 3'-end side, thus forming a stem-loop structure.

### (Step 3)

Next, starting from the 3'-end of this stem-loop structure (stem region B1), the synthesis of a new DNA strand starts using the single-stranded self as a template, and extends while stripping the third template nucleic acid synthesized from the Fw inner primer forming the double strand.

### (Step 4)

In addition, since the third template nucleic acid synthesized from the Fw inner primer, which has been made single-stranded by the above process, has complementary base sequences, that is, F1 and F1c and B1c and B1, at both ends thereof, it self-anneals to form a stem-loop structure. The structure of this third template nucleic acid is completely complementary to that of the second template nucleic acid, as described above.

### (Step 5)

In the structure obtained in step 4, similarly to the second template nucleic acid in step 1, a DNA strand is synthesized by an extension reaction starting from the 3'-end (stem region B1) and using the self as a template. Here, the stem-loop structure on the 5'-end side is stripped to form a single strand. Furthermore, since the third template nucleic acid has a single-stranded loop structure (structure composed of the base sequence BLc) on the 3'-end side, the base sequence B2 of the Bw inner primer can be annealed thereto, whereby a DNA strand is synthesized using the third template nucleic acid as a template by an extension reaction starting from the 3'-end of the Bw inner primer. Here, the extension reaction of a new DNA strand proceeds while stripping the existing DNA strand.

### (Step 6)

After step 5, the structure of the second template nucleic acid is obtained again by going through the same processes as steps 2 to 4.

### (Step 7)

In addition, in the new DNA strand obtained in step 3, the base sequence B2 of the Bw inner primer is annealed to the single-stranded loop structure (structure composed of the base sequence BLc), and a DNA strand is synthesized while stripping the double-stranded portion.

By repeating the above steps, it is possible to obtain various sizes of amplified products having a structure in which the base sequence Nc and its complementary strand and the base sequences complementary to each other (that is, F1 and F1c, and B1c and B1) are repeated on the same strand.

Usable examples of the complementary strand synthases used in the LAMP method include, but are not limited to, one or a combination of two or more of Bst DNA polymerase, Bca (exo-) DNA polymerase, Klenow fragment of E. coli DNA polymerase I, Csa DNA polymerase, and Bst 2.0 Warm Start DNA polymerase.

The reaction temperature in the LAMP method according to the present invention is not particularly limited, but is preferably 50 to 70°C, more preferably 60 to 70°C. Furthermore, the reaction time in the LAMP method according to the present invention is preferably 10 to 60 minutes, more preferably 15 to 40 minutes.

### (Detection Step)

The amplified product obtained in step C (in the present specification, referred to as "LAMP product" as the case may be) can be detected by a known method or a method based thereon as appropriate. As a result, the base sequence Nc or its complementary strand is detected, and since the base sequence Nc is the complementary strand of the target region N, this makes it possible to detect the target region N indirectly.

As the detection method, for example, the resulting LAMP product is intercalated with a fluorescent dye (such as ethidium bromide, Syber Green (registered trademark), or SYTO 63), and the fluorescence intensity can be measured to quantify the base sequence Nc or its complementary strand, obtaining the amount of the target region N. Moreover, the quantification can be performed simultaneously by performing the LAMP method and the detection step in real time, for example. Examples of such methods include a method that performs the LAMP method in the presence of a fluorescent dye, typified by the intercalation method (so-called Syber Green method); and a method that uses an oligonucleotide probe bound with a fluorescent dye (hereinafter simply referred to as "probe" as the case may be), typified by the double dye probe method (so-called TaqMan (registered trademark) probe method).

Among these, the nucleic acid amplification method of the present invention preferably further includes, after step C, a detection step of detecting the base sequence Nc (including the detection of the base sequence Nc by detecting a complementary strand of the base sequence Nc, same below) using a probe that hybridizes to at least part of the base sequence Nc or at least part of a complementary strand of the base sequence Nc (more preferably, a probe composed only of a base sequence that hybridizes to at least part of the base sequence Nc or at least part of a complementary strand of the base sequence Nc). By detecting these using a probe designed to hybridize on the base sequence Nc or a complementary strand of the base sequence Nc, it is possible to further reduce the detection of non-specific reactions caused by primer dimers.

A probe according to the detection method above can be appropriately designed based on the base sequence Nc or the base sequence of a complementary strand of the base sequence Nc, and the use of the following first probe or second probe is preferable because it makes it possible to further reduce the detection of non-specific reactions.

### [First Probe]

When the probe according to the detection method above is used as a probe that hybridizes to at least part of the base sequence Nc (referred to as the "first probe"), the length of the base sequence that hybridizes to the base sequence N3c among the base sequences Nc is preferably 5 bases long or less. As a result, specifically, the length of the base sequence that hybridizes to the base sequence N3c of the Bw adapter primer is also 5 bases long or less, and hybridization between the first probe and the Bw adapter primer is suppressed, so that in the case of simultaneously performing step C and the detection step (that is, when performing in real time), it is possible to further reduce non-specific amplification reactions due to such hybridization.

In the first probe, the length of the base sequence that hybridizes to the base sequence N3c is preferably 5 bases long or less, more preferably 4 bases long or less, and further preferably 3 bases long or less.

### [Second Probe]

Further, as another aspect according to the detection method above, when used as a probe that hybridizes to at least part of a complementary strand of the base sequence Nc (referred to as the "second probe"), preferably, the full-length base sequence is not contained entirely in one of the base sequence N3c and the base sequence N5c. As a result, the base sequence of the second probe is not contained in only one of the base sequence N3c of the Bw adapter primer and the complementary strand (N5c) of the base sequence N5' of the Fw adapter nucleotide, so that it is possible to detect only amplified products that perfectly match the LAMP products formed in accordance with principle, further reducing non-specific detection.

As the second probe, the base sequence contained in the base sequence N3c and the base sequence contained in the base sequence N5c are each preferably 90% or less, more preferably 80% or less, and further preferably 70% or less of the full length of the probe.

The probes according to the detection method above preferably specifically hybridize to each hybridization target region under normal hybridization conditions, preferably stringent hybridization conditions, and for example, it is preferable to satisfy the above hybridization conditions, and it is possible to design by a conventionally known method or a method based thereon based on the base sequence information of each hybridization target region.

The chain length of the probes according to the detection method above is at least 5 bases. It is usually 5 to 100 bases, preferably 5 to 30 bases. The probes can be synthesized, for example, using a commercially available oligonucleotide synthesizer. In addition, the probes do not have to be composed only of natural nucleotides (deoxyribonucleotides and/or ribonucleotides), and may be partially or wholly composed of the non-natural nucleotides, for example.

Moreover, the probe is preferably labeled with a labeling substance as appropriate. As a result, a signal corresponding to the labeling substance can be detected as an indicator of the base sequence Nc or its complementary strand. The "signal" includes coloration (color development), reflected light, luminescence, quenching, fluorescence, radiation from radioactive isotopes, and the like, as well as those that can be confirmed with the naked eye and those that can be confirmed by measuring methods and devices according to the type of the signal.

Examples of the labeling substances include fluorescent substances such as FITC, FAM, DEAC, R6G, TexRed, TAMRA, Pacific Blue, Cy5, and BODIPY FL; enzymes such as β-D-glucosidase, luciferase, and HRP; radioactive isotopes such as ³H, ¹⁴C, ³²P, ³⁵S, and ¹²³I; affinity substances such as biotin and streptavidin; and luminescent substances such as luminol, luciferin, and lucigenin.

### <Primer and Adaptor Set, Probe, Kit>

The present invention also provides use of a primer and adapter set, and use of a kit in the nucleic acid amplification method of the present invention described above.

The primer and adapter set to be used in the nucleic acid amplification method of the present invention comprises:
a Bw adapter primer comprising structure (a):

   5'-B1c-BL-B1-N3c-3' ... (a)

   and
an Fw adapter nucleotide comprising structure (c):

   5'-F1c-FL-F1-N5'-3' ... (c)
and having an extension-inhibiting modification at the 3'-end. The primer and adapter set for the nucleic acid amplification method of the present invention preferably further comprises:
   an Fw inner primer comprising structure (e):

      5'-F1c-F2-3' ... (e)

      and
   a Bw inner primer comprising structure (f):

      5'-B1c-B2-3' ... (f).

In addition, the probe for the nucleic acid amplification method as disclosed herein is
a probe for use in the nucleic acid amplification method as disclosed herein, which hybridizes to at least part of the base sequence Nc or at least part of a complementary strand of the base sequence Nc.

Furthermore, the kit to be used in the nucleic acid amplification method of the present invention comprises the primer and adapter set for the nucleic acid amplification method of the present invention, and the probe as mentioned above.

In the use of a primer and adaptor set and use of a kit in the nucleic acid amplification method of the present invention, the Bw adapter primer, the Fw adapter nucleotide, the Fw inner primer, the Bw inner primer, and the probe, including their preferred embodiments, are each as described in the nucleic acid amplification method of the present invention.

In the use of a primer and adaptor set in the nucleic acid amplification method of the present invention, the primer, nucleotide, and probe may each comprise an additional component such as a buffer, stabilizer, preservative, or antiseptic.

In addition, the kit to be used in the nucleic acid amplification method of the present invention may further include reagents and diluents for extracting and purifying the target nucleic acid; reagents such as enzymes, buffer solutions, and pH adjusters necessary for various enzymatic reactions; standard nucleic acids; instructions for use, and the like.

### [Examples]

### (Test Example 1) Effects of the stem-loop structure of the adapter primer on the reaction

### (1) Reverse transcription reaction and extension reaction (template nucleic acid synthesis)

The reverse transcription reaction and extension reaction were performed as follows. In a buffer containing KCl, Tween, MgSO₄ and dNTPs, 125 nM Bw adapter primer, 5 nM Fw adapter nucleotide, 0.9 U AMV (Roche), and 3.6 U Bst 2.0 Warm Start DNA Polymerase (manufactured by New England Biolabs) at final concentrations were mixed, and the miRNA miR-21-5p (whose base sequence is set forth in SEQ ID NO: 3) or Let-7a-5p (whose base sequence is set forth in SEQ ID NO: 6) was added as the target nucleic acid at 10⁹ copies each or was not added (NC), and the reactions were performed at 42°C, thereby synthesizing a template nucleic acid. Table 1 below shows the combinations of Bw adapter primer and Fw adapter nucleotide used for miR-21-5p and Let-7a-5p. In Table 1, "CT" of the Bw adapter primer represents a primer with a stem-loop structure, and as controls, "ST1" and "ST2" each represent a primer that does not form a stem-loop structure (the region corresponding to B1c of the Bw adapter primer is replaced with a sequence that does not form a base pair with the region corresponding to B1), and "D1" represents a primer that has deleted the region corresponding to B1c of the Bw adapter primer. In addition, in the sequence of each Fw adapter nucleotide in Table 1, each block separated by "-" represents the base sequence corresponding to F1c, F2, FS, F1, and N5' in order from the 5'-end, and in the sequence of each Bw adapter primer, each block separated by "-" represents the base sequence corresponding to B1c, B2, BS, B1, and N3c in order from the 5'-end.

**[Table 1]**

| Name | | Sequence | SEQ ID NO |
|---|---|---|---|
| miR-21-5p | | | |
| miR-21-5p | | 5'-UAGCUUAUCAGACUGAUGUUGA-3' | 3 |
| Fw Adapter Nucleotide | | 5'-TAACGAGCGGCTTCC-CCAAACCGTCTTTCACCAC- ATCGCATTATATCGTTCTTCTAAATCAAAGTA-GGAAGCCGCTCGTTA-TAGCTTATCAG-PHO-3' | 1 |
| Bw Adapter Primer | CT | 5'-GCAACTCAATCCGTTGA-AAAGAGCGGTATCCCCAT- CATCATCGACAAGGTTTATAATTTGGGCAGCGACA-TCAACGGATTGAGTTGC-TCAACATCAGT-3' | 2 |
| | ST1 | 5'-TCAACGGATTGAGTTGC-AAAGAGCGGTATCCCCAT- CATCATCGACAAGGTTTATAATTTGGGCAGCGACA-TCAACGGATTGAGTTGC-TCAACATCAGT-3' | 7 |
| | ST2 | 5'-CATCATCGACAAGGTTT-AAAGAGCGGTATCCCCAT- CATCATCGACAAGGTTTATAATTTGGGCAGCGACA-TCAACGGATTGAGTTGC-TCAACATCAGT-3' | 8 |

| Let-7a-5p | | | |
|---|---|---|---|
| Let-7a-5p | | 5'-UGAGGUAGUAGGUUGUAUAGUU-3' | 6 |
| Fw Adapter Nucleotide | | 5'-TAACGAGCGGCTTCC-CCAAACCGTCTTTCACCAC- ATCGCATTATATCGTTCTTCTAAATCAAAGTA-GGAAGCCGCTCGTTA-TGAGGTAGTAG-PHO-3' | 4 |
| Bw Adapter Primer | CT | 5'-GCAACTCAATCCGTTGA-AAAGAGCGGTATCCCCAT- CATCATCGACAAGGTTTATAATTTGGGCAGCGACA-TCAACGGATTGAGTTGC-AACTATACAAC-3' | 5 |
| | D1 | 5'-AAAGAGCGGTATCCCCAT- CATCATCGACAAGGTTTATAATTTGGGCAGCGACAwTCAACGGATTGAGTTGC-AACTATACAAC-3' | 9 |
| | ST1 | 5'-TCAACGGATTGAGTTGC-AAAGAGCGGTATCCCCAT- CATCATCGACAAGGTTTATAATTTGGGCAGCGACA-TCAACGGATTGAGTTGC-AACTATACAAC-3' | 10 |
| | ST2 | 5'-CATCATCGACAAGGTTT-AAAGAGCGGTATCCCCAT- CATCATCGACAAGGTTTATAATTTGGGCAGCGACA-TCAACGGATTGAGTTGC-AACTATACAAC-3' | 11 |

| | | | |
|---|---|---|---|
| * PHO: phosphorylation | | | |

### (2) PCR amplification reaction and fluorescence detection

To the reaction solution after the above reverse transcription reaction and extension reaction, 1 µM PCR-F-primer (whose base sequence is set forth in SEQ ID NO: 12), 1 µM PCR-B-primer (whose base sequence is set forth in SEQ ID NO: 13), SYTO 63 (manufactured by Thermo Fisher Scientific), and 0.03 U/µM Takara Ex Taq (manufactured by Takara Bio) at final concentrations were added to prepare a PCR amplification reaction solution. Each PCR primer (F-primer/B-primer) was designed on the sequence of each loop region of the Bw adapter primer and Fw adapter nucleotide. The PCR amplification reaction was performed by maintaining the above PCR amplification reaction solution at 95°C for 10 seconds and then performing [95°C for 15 seconds and 60°C for 60 seconds] × 40 cycles. The amount of PCR product was measured in real time by detecting fluorescence intensity under the conditions of an excitation wavelength of 618 nm and a measurement wavelength of 660 nm using LightCycler (registered trademark) 480 Instrument II (manufactured by Roche).

Each Ct value was obtained from the fluorescence intensity-reaction time curves obtained by the measurements, and the difference with the Ct value of NC (Ct value of NC - Ct value of 109 copies of target nucleic acid) was calculated as ΔCt. Tables 2 and 3 below show the results.

**[Table 2]**

| miR-21-5p | | | |
|---|---|---|---|
| Bw Adapter Primer | CT | ST1 | ST2 |
| ΔCt Value | 8.8 | 4.4 | 1.1 |

**[Table 3]**

| Let-7a-5p | | | | |
|---|---|---|---|---|
| Bw Adapter Primer | CT | D1 | ST1 | ST2 |
| ΔCt Value | 7.2 | 2.5 | 4.7 | 1.8 |

As shown in Tables 2 and 3, in both cases of using miR-21-5p and Let-7a-5p as the target nucleic acid, the ΔCT values when using ST1, ST2, and D1 were decreased (that is, the Ct values were increased) compared to the ΔCT values when using CT as the Bw adapter primer. These results suggest that by changing the structure of the Bw adapter primer from a linear structure to a stem-loop structure and introducing a stem-loop structure into the template nucleic acid, the amplification cycle is accelerated and the reactivity is improved.

### (Test Example 2) Verification of heat treatment effects after extension reaction

### (1) Reverse transcription reaction and extension reaction (template nucleic acid synthesis)

The reverse transcription reaction and extension reaction were performed as follows. In a buffer containing KCl, Tween, MgSO₄ and dNTPs, 5 nM Bw adapter primer (SEQ ID NOs: 1 and 4), 5 nM adapter nucleotide as the Fw adapter nucleotide whose 3'-end was not phosphorylated in the base sequences set forth in SEQ ID NOs: 1 and 4, 0.9 U AMV (Roche), and 3.6 U Bst 2.0 Warm Start DNA Polymerase (manufactured by New England Biolabs) at final concentrations were mixed, and miR-21-5p (SEQ ID NO: 3) or Let-7a-5p (SEQ ID NO: 6) was added at 10⁷ copies, 10⁹ copies, or 10¹¹ copies each or was not added (NC), and the reactions were performed at 42°C, thereby synthesizing a template nucleic acid. After that, heat treatment was performed by heating at 95°C for 5 minutes. In addition, as a control, the reverse transcription reaction and extension reaction were performed in the same manner, and then the heat treatment was not performed.

### (2) LAMP amplification reaction and fluorescence detection

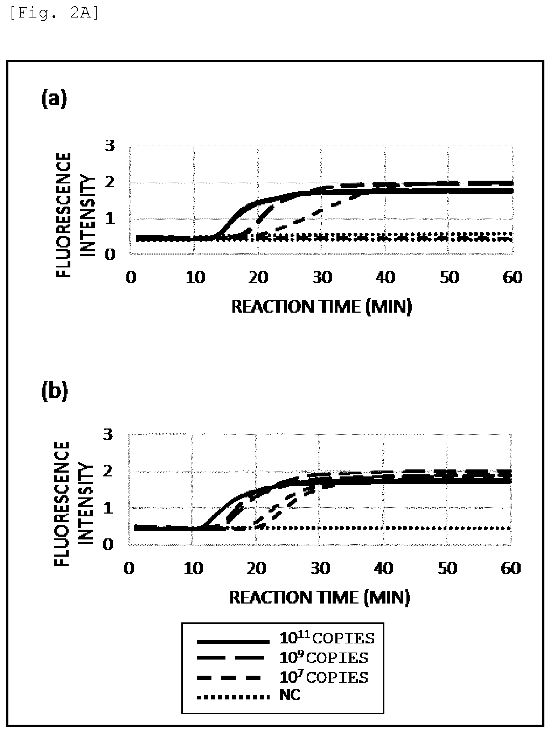

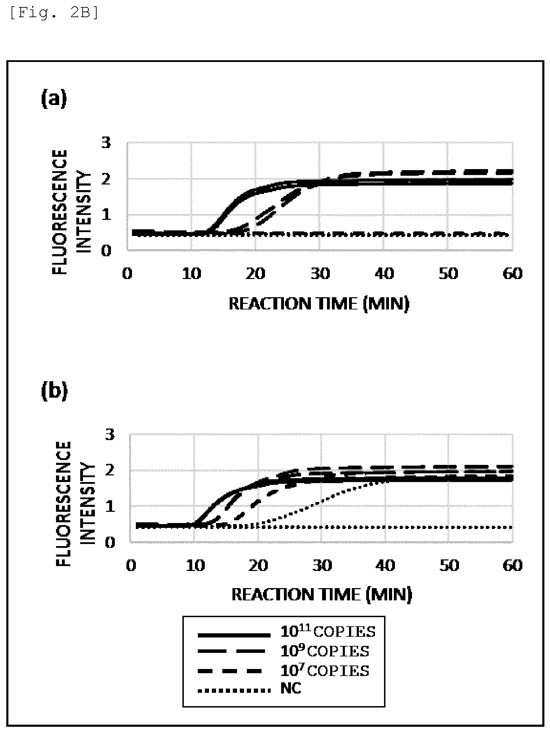

To the reaction solution after the above heat treatment (with heat treatment) or after the reverse transcription reaction and extension reaction (without heat treatment), 0.8 µM Fw inner primer (whose base sequence is set forth in SEQ ID NO: 14), 0.8 µM Bw inner primer (whose base sequence is set forth in SEQ ID NO: 15), SYTO 63 (manufactured by Thermo Fisher Scientific), and 2.4 U Bst 2.0 Warm Start DNA Polymerase at final concentrations were added to prepare a LAMP amplification reaction solution. The reaction solution was subjected to the LAMP amplification reaction at 62°C for 60 minutes. The amount of LAMP product was measured in real time by detecting fluorescence intensity under the conditions of an excitation wavelength of 618 nm and a measurement wavelength of 660 nm using LightCycler (registered trademark) 480 Instrument II (manufactured by Roche). Figs. 2A and 2B show fluorescence intensity-reaction time curves obtained by the measurements. Fig. 2A shows the results using miR-21-5p as the target nucleic acid, and Fig. 2B shows the results using Let-7a-5p as the target nucleic acid, and in Figs. 2A and 2B, (a) shows the results without heat treatment, and (b) shows the results with heat treatment.

As shown in Figs. 2A and 2B, in both cases of using miR-21-5p and Let-7a-5p as the target nucleic acid, it was confirmed that (a) without heat treatment, as the copy number of the target nucleic acid decreased, the rise of the fluorescence intensity-reaction time curve slowed down. Meanwhile, (b) with heat treatment, a sufficient rise of the curve was confirmed even when the copy number of the target nucleic acid was small. However, when Let-7a-5p was used, the rise of the curve was faster even in NC with heat treatment (Fig. 2B(b)). From these results, it was confirmed that the heat treatment after complementary strand synthesis contributed to the enhancement of the detection sensitivity of amplification products.

### (Test Example 3) Verification of 3'-end phosphorylation effects of Fw adapter nucleotide (3'-end extension-inhibiting modification effects)

In Test Example 2, non-specific amplification reaction in NC was confirmed when Let-7a-5p was used. Thus, for the purpose of suppressing such non-specific amplification reaction, the 3'-end phosphorylation effects of the Fw adapter nucleotide were verified.

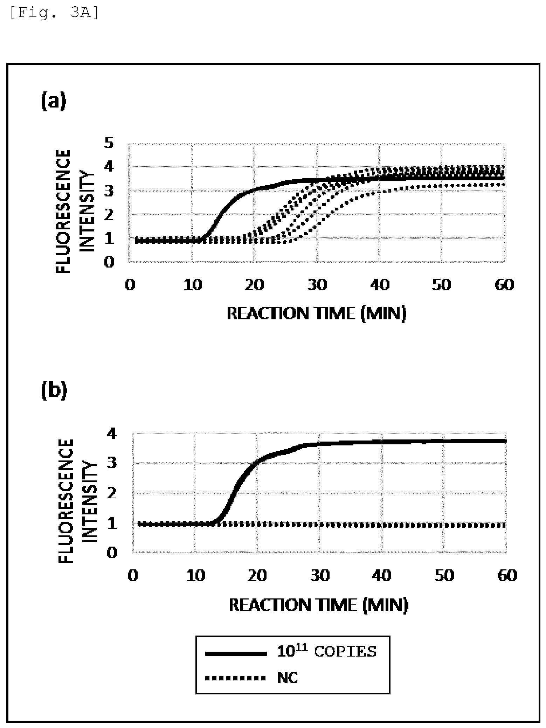

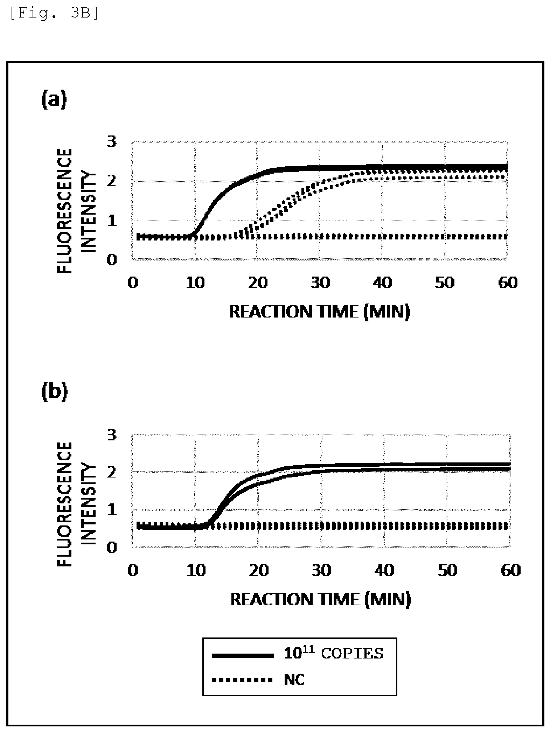

Specifically, first, in the same manner as in Test Example 2 (1), miR-21-5p (SEQ ID NO: 3) or Let-7a-5p (SEQ ID NO: 6) was added at 10¹¹ copies each or was not added (NC) to perform the reverse transcription reaction and extension reaction (without phosphorylation). In addition, the reverse transcription reaction and extension reaction were performed (with phosphorylation) except for using, as Fw adapter nucleotides, adapter nucleotides in which the 3'-ends of the base sequences set forth in Table 1 and SEQ ID NOs: 1 and 4 were phosphorylated. Then, after each reverse transcription reaction and extension reaction, heat treatment was performed by heating at 95°C for 5 minutes. Then, LAMP amplification reaction and fluorescence detection were performed in the same manner as in Test Example 2 (2) to obtain each fluorescence intensity-reaction time curve. Figs. 3A and 3B show the obtained fluorescence intensity-reaction time curves. Fig. 3A shows the results using miR-21-5p as the target nucleic acid, and Fig. 3B shows the results using Let-7a-5p as the target nucleic acid, and in Figs. 3A and 3B, (a) shows the results without phosphorylation, and (b) shows the results with phosphorylation.

As shown in Figs. 3A and 3B, in both cases of using miR-21-5p and Let-7a-5p as the target nucleic acid, it was confirmed that (a) without phosphorylation at the 3'-end of the Fw adapter nucleotide, a rise in the fluorescence intensity-reaction time curve was confirmed for NC when the reaction time was between 20 minutes and 30 minutes. Meanwhile, with phosphorylation (b), no rise in NC was confirmed in any case. Moreover, there was almost no difference in the rise time for 10¹¹ copies of the target nucleic acid between the presence and absence of phosphorylation of the 3'-end of the Fw adapter nucleotide. These results confirmed that 3'-end phosphorylation of Fw adapter nucleotides could suppress non-specific amplification reactions without affecting detection in the presence of the target nucleic acid.

### (Test Example 4) Fluorescence quenching probe detection

### (1) Reverse transcription reaction and extension reaction (template nucleic acid synthesis)

In the same manner as in Test Example 2 (1), miR-21-5p (SEQ ID NO: 3) or Let-7a-5p (SEQ ID NO: 6) was added at 10¹¹ copies or was not added (NC) to perform the reverse transcription reaction and extension reaction and synthesize template nucleic acids, except for setting Bw adapter primers (SEQ ID NOs: 2 and 5) at a final concentration of 125 nM, using, as Fw adapter nucleotides, adapter nucleotides in which the 3'-ends of the base sequences set forth in Table 1 and SEQ ID NOs: 1 and 4 were phosphorylated, and setting the Fw adapter nucleotides (SEQ ID NOs: 1 and 4) to a final concentration of 50 nM. After that, heat treatment was performed by heating at 95°C for 5 minutes.

### (2) LAMP amplification reaction

To the reaction solution after the above heat treatment, 0.8 µM Fw inner primer (SEQ ID NO: 14), 0.8 µM Bw inner primer (SEQ ID NO: 15), 2.4 U Bst 2.0 Warm Start DNA Polymerase, and 0.04 µM fluorescence quenching probe (fluorochrome: BODIPY FL) at final concentrations were added to prepare a LAMP amplification reaction solution. Table 4 below shows the fluorescence quenching probes used for miR-21-5p and Let-7a-5p. In Table 4, the "miR-21-5p Region Design Probe" is a probe (whose base sequence is set forth in SEQ ID NO: 16) designed to hybridize to the base sequence (SEQ ID NO: 3) of the target nucleic acid miR-21-5p, and the "Let-7a-5p Region Design Probe" is a probe (whose base sequence is set forth in SEQ ID NO: 17) designed to hybridize to the base sequence (SEQ ID NO: 6) of the target nucleic acid Let-7a-5p, and as their control, the "Loop Region Design Probe" is a probe (whose base sequence is set forth in SEQ ID NO: 18) designed to hybridize to the complementary strand of the loop region (BL, especially the region corresponding to B2) of each Bw adapter primer (SEQ ID NOs: 2 and 5). The LAMP amplification reaction was performed at 62°C for 60 minutes using the LAMP amplification reaction solution.

**[Table 4]**

| Name | Sequence | SEQ ID NO |
|---|---|---|
| miR-21-5p | 5'-**BP**-CAGTCTGATAA-**PHO**-3' | 16 |
| Region Design Probe | | |
| Let-7a-5p | 5'-**BP**-CAACCTACTAC-**PHO**-3' | 17 |
| Region Design Probe | | |
| Loop Region Design Probe | 5' -CGACAAGGTTTATAATTTGGGCAGCIAC-**BP**-3' | 18 |

| | | |
|---|---|---|
| * **BP:** BODIPY FL * **PHO:** phosphorylation * I: Inosine | | |

### (3) Melting curve analysis

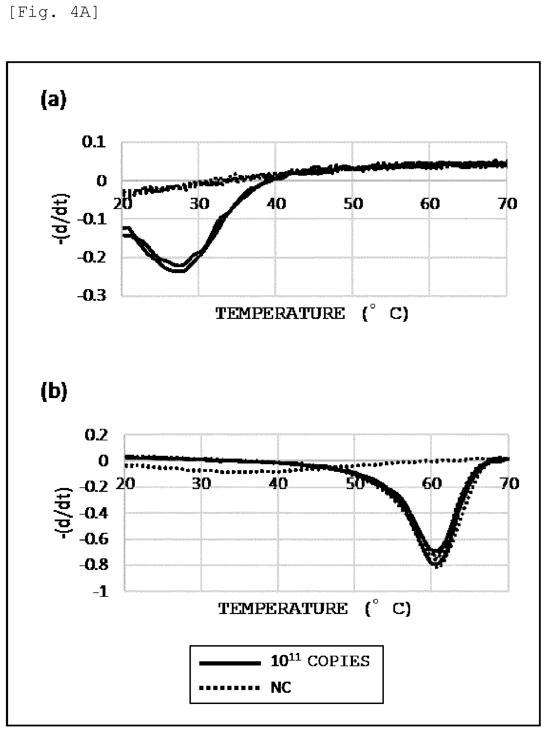

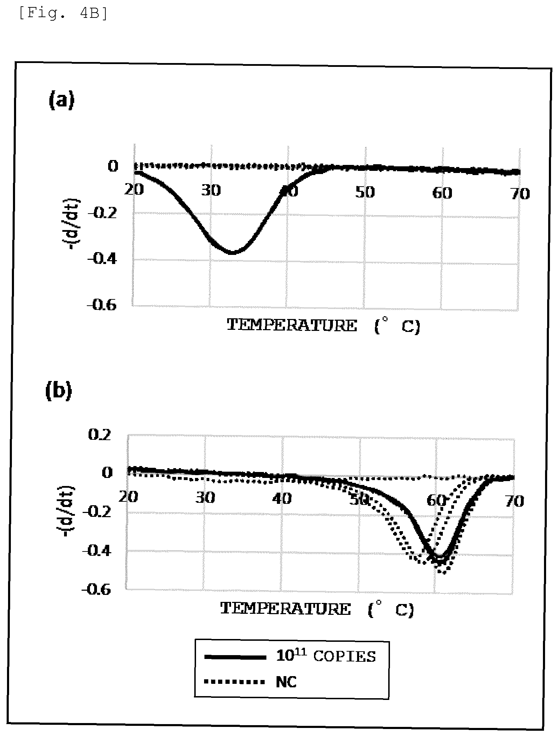

After the LAMP amplification reaction, the reaction solution was heated at 95°C for 5 minutes for thermal denaturation. Then, while lowering the temperature to 20°C, fluorescence intensity was measured using LightCycler (registered trademark) 480 Instrument II (manufactured by Roche) under the conditions of an excitation wavelength of 465 nm and a measurement wavelength of 510 nm. Thereby, melting curves were obtained. Figs. 4A and 4B show the melting curves obtained by the measurements. Fig. 4A shows the results using miR-21-5p as the target nucleic acid, and Fig. 4B shows the results using Let-7a-5p as the target nucleic acid, and in Figs. 4A and 4B, (a) shows the results using a probe (SEQ ID NO: 16 or SEQ ID NO: 17) designed to hybridize to the base sequence of each target nucleic acid, and (b) shows the results using a probe (SEQ ID NO: 18) designed to hybridize to the complementary strand of the loop region of each Bw adapter primer.

As shown in Figs. 4A and 4B, in both cases of using miR-21-5p and Let-7a-5p as the target nucleic acid, in case (b) of using a probe designed to hybridize to the complementary strand of the loop region of the Bw adapter primer, quenching was observed not only in the presence of target nucleic acid but also in NC. Meanwhile, in case (a) of using a probe designed to hybridize to the base sequence of the target nucleic acid, quenching was observed in the presence of the target nucleic acid, but not in NC. From these results, it has been confirmed that in detecting a LAMP amplification product with a probe, non-specific quenching is suppressed by designing the probe so as to hybridize to the base sequence of the target nucleic acid, that is, on the region of the template nucleic acid corresponding to the target nucleic acid, enabling more specific detection.

### (Test Example 5) Verification of probe design area

In Test Example 4, designing the probe on the region corresponding to the target nucleic acid of the template nucleic acid enabled more specific detection, so that the design region of the probe was verified in more detail.

Specifically, first, in the same manner as in Test Example 2 (1), miR-21-5p (SEQ ID NO: 3) was added at 10⁷ copies each or was not added (NC) to perform the reverse transcription reaction and extension reaction and synthesize template nucleic acids, except for setting a Bw adapter primer (SEQ ID NO: 2) at a final concentration of 125 nM, using, as an Fw adapter nucleotide, an adapter nucleotide in which the 3'-end of the base sequence set forth in Table 1 and SEQ ID NO: 1 was phosphorylated, and setting the Fw adapter nucleotide (SEQ ID NO: 1) to a final concentration of 50 nM. After that, heat treatment was performed by heating at 95°C for 5 minutes. Next, a LAMP amplification reaction was performed in the same manner as in Test Example 4 (2), except for using the fluorescence quenching probes shown in Table 5 below. In Table 5, "miRNA21Probe-1" to "miRNA21Probe-7" are probes designed to hybridize to the base sequence (SEQ ID NO: 3) of the target nucleic acid miR-21-5p or its complementary strand, and to have different numbers of bases to hybridize (the base sequences of Probe-2 to 7 are set forth in SEQ ID NOs: 19 to 24), and "miRNA21Probe-8" is the same as the above "miR-21-5p Region Design Probe (SEQ ID NO: 16)". Table 5 also shows the base sequences of the sense strand and antisense strand of the target nucleic acid miR-21-5p, and the sequences corresponding to the base sequence N3c of the Bw adapter primer or its complementary strand are underlined.

**[Table 5]**

| Name | Sequence | SEQ ID NO |
|---|---|---|
| miR-21-5p | 5'-UAGCUUAUCAGACUGAUGUUGA-3' | 3 |
| Sense Strand | | |
| miRNA21Probe-1 | 5'-TAGCTTATC-**BP**-3' | - |
| miRNA21Probe-2 | 5'-**BP**-CTTATCAGAC-**PHO**-3' | 19 |
| miRNA21Probe-3 | 5'-**BP**-CTTATCAGACT-**PHO**-3' | 20 |
| miRNA21Probe-4 | 5'-**BP**-CTTATCAGACTGA-**PHO**-3' | 21 |
| miRNA21Probe-5 | 5'-**BP**-CTTATCAGACTGATGT-**PHO**-3' | 22 |
| miRNA21Probe-6 | 5'-**BP**-CTTATCAGACTGATGTTGA-**PHO**-3' | 23 |
| miR-21-5p | 5'-UCAACAUCAGUCUGAUAAGCUA-3' | |
| Antisense Strand | | |
| miRNA21Probe-7 | 5'-**BP**-CAACATCAGT-**PHO**-3' | 24 |
| miRNA21Probe-8 | 5'-**BP**-CAGTCTGATAA-**PHO**-3' | 16 |

| | | |
|---|---|---|
| * **BP:** BODIPY FL * **PHO:** phosphorylation | | |

### (3) Melting curve analysis

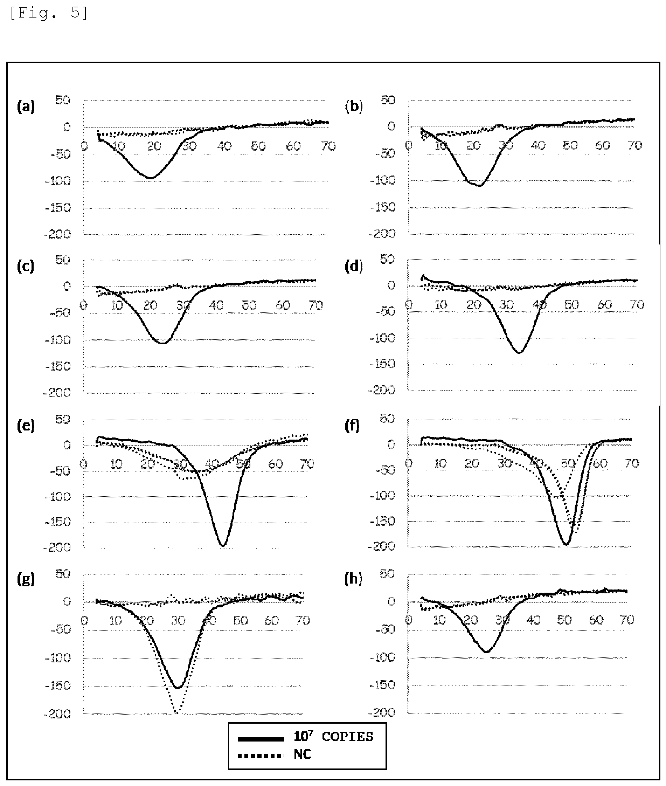

After the LAMP amplification reaction, the reaction solution was heated at 95°C for 5 minutes for thermal denaturation. Then, while lowering the temperature to 4°C, fluorescence intensity was measured using CFX96 Touch Deep Well Real-Time PCR Analysis System (manufactured by Bio-Rad) under the conditions of an excitation wavelength of 450 to 490 nm and a measurement wavelength of 515 to 530 nm. Thereby, melting curves were obtained. Fig. 5 shows the melting curves obtained by the measurements. In Fig. 5, the vertical axis of each curve indicates -(d/dt), and the horizontal axis indicates temperature (°C). The results are such that (a) is with use of miRNA21Probe-1, (b) is with use of miRNA21Probe-2, (c) is with use of miRNA21Probe-3, (d) is with use of miRNA21Probe-4, (e) is with use of miRNA21Probe-5, (f) is with use of miRNA21Probe-6, (g) is with use of miRNA21Probe-7, and (h) is with use of miRNA21Probe-8.

As shown in Fig. 5, when designing a probe for the sense strand of the target nucleic acid (when designing a probe so as to hybridize to the antisense strand of the target nucleic acid), that is, when designing a probe so as to hybridize to the base sequence (base sequence Nc) complementary to the sense strand on a template nucleic acid ((a) to (f)), quenching was observed only in the presence of the target nucleic acid for miRNA21Probe-1 to 4 ((a) to (d)), and quenching was also observed in NC for miRNA21Probe-5 and 6 ((e) and (f)). It was considered that in miRNA21Probe-5 and 6, the Bw adapter primer interfered with the probe and caused non-specific amplification.

Meanwhile, when designing a probe for the antisense strand of the target nucleic acid (when designing a probe so as to hybridize to the base sequence of the sense strand of the target nucleic acid), that is, when designing a probe so as to hybridize to a complementary strand of the base sequence Nc on a template nucleic acid ((g) and (h)), quenching was also observed in NC for miRNA21Probe-7 (g), and quenching was observed only in the presence of the target nucleic acid for miRNA21Probe-8.

It has been confirmed from these results that non-specific quenching is suppressed and more specific detection becomes possible by designing so that the length bondable to the Bw adapter primer (the length of the base sequence that hybridizes to N3c) is short when designing a probe for the sense strand of the target nucleic acid, that is, when designing a probe so as to hybridize to the base sequence Nc of the template nucleic acid, and by designing so that at least the entire sequence of the probe is not contained in the base sequence of the Bw adapter primer (especially, the entire sequence of the probe does not hybridize to the complementary strand of the base sequence N3c) when designing a probe for the antisense strand of the target nucleic acid, that is, when designing a probe so as to hybridize to a complementary strand of the base sequence Nc of the template nucleic acid.

### [Industrial Applicability]

As described above, the present invention makes it possible to provide a nucleic acid amplification method capable of simply and specifically amplifying a nucleic acid using a short-chain nucleic acid such as fragmented DNA or miRNA as a template, as well as a primer set, a probe, and a kit for the nucleic acid amplification method used therefor.

### [Reference Signs List]

11: target nucleic acid (target region N)
20: Bw adapter primer
21: stem region B1c
221: base sequence B2
222: spacer BS
23: stem region B1
24: annealing region N3c
25: base sequence N5c
30: Fw adapter nucleotide
31: stem region F1c
321: base sequence F2
322: spacer FS
33: stem region F1
34: annealing region N5'
361: base sequence F2c complementary to base sequence F2
362: base sequence FSc complementary to spacer FS
40: first template nucleic acid
50: second template nucleic acid
60: Fw inner primer
101: reverse transcriptase
102: DNA polymerase

### [Sequence Listing Free Text]

SEQ ID NO: 1
   <223> miR-21-5p Fw adapter nucleotide
SEQ ID NO: 2
   <223> miR-21-5p Bw adapter primer CT
SEQ ID NO: 4
   <223> Let-7a-5p Fw adapter nucleotide
SEQ ID NO: 5
   <223> Let-7a-5p Bw adapter primer CT
SEQ ID NO: 7
   <223> miR-21-5p Bw adapter primer ST1
SEQ ID NO: 8
   <223> miR-21-5p Bw adapter primer ST2
SEQ ID NO: 9
   <223> Let-7a-5p Bw adapter primer D1
SEQ ID NO: 10
   <223> Let-7a-5p Bw adapter primer ST1
SEQ ID NO: 11
   <223> Let-7a-5p Bw adapter primer ST2
SEQ ID NO: 12
   <223> PCR-F-primer
SEQ ID NO: 13
   <223> PCR-B-primer
SEQ ID NO: 14
   <223> Fw inner primer
SEQ ID NO: 15
   <223> Bw inner primer
SEQ ID NO: 16
   <223> miR-21-5p region design probe
SEQ ID NO: 17
   <223> Let-7a-5pregion design probe
SEQ ID NO: 18
   <223> loop region design probe
   <223> n indicates inosine

## Claims

1. A method for amplifying nucleic acids, comprising:
step A including annealing a Bw adapter primer comprising the following structure (a):
5'-B1c-BL-B1-N3c-3' ... (a)
[Here, N3c represents an annealing region composed of a base sequence complementary to a base sequence on a 3'-end side of a target region of a target nucleic acid, BL represents a loop region comprising base sequence B2, and B1c and B1 represent stem regions comprising mutually complementary base sequences and capable of forming a double strand.]
and the target region, synthesizing a base sequence complementary to a base sequence on a 5'-end side of the target region starting from a 3'-end of the Bw adapter primer, and obtaining a first template nucleic acid comprising the following structure (b):
5'-B1c-BL-B1-N3c-N5c-3' ... (b)
[Here, N5c represents the base sequence complementary to the base sequence on the 5'-end side of the target region, and a base sequence comprising N3c and N5c represents base sequence Nc complementary to the target region.]
in which a stem-loop structure is added to a 5'-end of a complementary strand of the target region;
step B including annealing an Fw adapter nucleotide comprising the following structure (c):
5'-F1c-FL-F1-N5'-3' ... (c)
[Here, N5' represents an annealing region composed of a base sequence complementary to base sequence N5c of the first template nucleic acid, FL represents a loop region comprising base sequence F2, and F1c and F1 represent stem regions comprising mutually complementary base sequences and capable of forming a double strand.]
and having an extension-inhibiting modification at a 3'-end
and the first template nucleic acid, synthesizing a complementary strand of the Fw adapter nucleotide starting from a 3'-end of the first template nucleic acid, and obtaining a second template nucleic acid comprising the following structure (d):
5'-B1c-BL-B1-N3c-N5c-F1c-FLc-F1-3' ... (d)
[Here, FLc represents a base sequence complementary to FL of the Fw adapter nucleotide.]
in which a stem-loop structure is added to a 3'-end of the first template nucleic acid; and
step C including amplifying base sequence Nc of the second template nucleic acid by a LAMP method by using an Fw inner primer comprising the following structure (e):
5'-F1c-F2-3' ... (e)
and a Bw inner primer comprising the following structure (f):
5'-B1c-B2-3' ... (f)
with the second template nucleic acid as a template.

2. The nucleic acid amplification method according to claim 1, wherein a length of the base sequence Nc is 10 to 100 bases long.

3. The nucleic acid amplification method according to claim 1 or 2, wherein the target nucleic acid is miRNA.

4. The nucleic acid amplification method according to any one of claims 1 to 3, wherein step A and step B proceed in the same reaction system.

5. The nucleic acid amplification method according to any one of claims 1 to 4, further comprising: a heat treatment step of heating the reaction product of step B to 85°C or higher after step B and before step C.

6. The nucleic acid amplification method according to any one of claims 1 to 5, further comprising: after step C, a detection step of detecting the base sequence Nc using a probe that hybridizes to at least part of the base sequence Nc or at least part of a complementary strand of the base sequence Nc.

7. The nucleic acid amplification method according to claim 6, wherein in the probe, a length of a base sequence that hybridizes to the base sequence N3c is 5 bases long or less.

8. The nucleic acid amplification method according to claim 6, wherein a full-length base sequence of the probe is not contained entirely in one of the base sequence N3c and the base sequence N5c.

9. Use of a primer and adapter set in the nucleic acid amplification method according to any one of claims 1 to 8, wherein the primer and adapter set comprises:
a Bw adapter primer comprising the following structure (a):
5'-B1c-BL-B1-N3c-3' ... (a)
[Here, N3c represents an annealing region composed of a base sequence complementary to a base sequence on a 3'-end side of a target region of a target nucleic acid, BL represents a loop region comprising base sequence B2, and B1c and B1 represent stem regions comprising mutually complementary base sequences and capable of forming a double strand.]
and an Fw adapter nucleotide comprising the following structure (c):
5'-F1c-FL-F1-N5'-3' ... (c)
[Here, N5' represents an annealing region composed of a base sequence complementary to base sequence N5c of the first template nucleic acid, FL represents a loop region comprising base sequence F2, and F1c and F1 represent stem regions comprising mutually complementary base sequences and capable of forming a double strand.]
and having an extension-inhibiting modification at the 3'-end.

10. The use according to claim 9, wherein the primer and adapter set further comprises:
an Fw inner primer comprising the following structure (e):
5'-F1c-F2-3' ... (e)
and
a Bw inner primer comprising the following structure (f):
5'-B1c-B2-3' ... (f).

11. Use of a kit in the nucleic acid amplification method according to any one of claims 1 to 8, wherein the kit comprises:
(i) the primer and adapter set according to claim 9 or 10; and
(ii) a probe that hybridizes to at least part of the base sequence Nc or at least part of a complementary strand of the base sequence Nc.

12. The use according to claim 11, wherein in the probe, a length of a base sequence that hybridizes to the base sequence N3c is 5 bases long or less.

13. The use according to claim 11, wherein a full-length base sequence of the probe is not contained entirely in one of the base sequence N3c and the base sequence N5c.

## Patentansprüche

1. Verfahren zum Amplifizieren von Nukleinsäuren, umfassend:
Schritt A, der das Schmelzen eines Bw-Adapterprimers, umfassend die folgende Struktur (a):
5'-B1c-BL-B1-N3c-3' ... (a)
[wobei N3c eine Schmelzregion darstellt, die aus einer Basensequenz besteht, die komplementär zu einer Basensequenz an einem 3'-Ende einer Zielregion einer Zielnukleinsäure ist, BL eine Schleifenregion darstellt, die die Basensequenz B2 umfasst, und B1c und B1 Stammregionen darstellen, die zueinander komplementäre Basensequenzen umfassen und in der Lage sind, einen Doppelstrang auszubilden.]
und der Zielregion, das Synthetisieren einer Basensequenz, die komplementär zu einer Basensequenz an einem 5'-Ende der Zielregion ist, ausgehend von einem 3'-Ende des Bw-Adapterprimers, und das Erhalten einer ersten Matrizennukleinsäure beinhaltet, die die folgende Struktur (b) umfasst:
5'-B1c-BL-B1-N3c-N5c-3' ... (b)
[wobei N5c die Basensequenz, die komplementär zu der Basensequenz an dem 5'-Ende der Zielregion ist, und eine Basensequenz, die N3c und N5c umfasst, und eine Basensequenz Nc darstellt, die komplementär zu der Zielregion ist.]
in dem eine Stamm-Schleife-Struktur an ein 5'-Ende eines komplementären Strangs der Zielregion hinzugefügt wird;
Schritt B, der das Anlagern eines Fw-Adapter-Nukleotids, umfassend die folgende Struktur (c):
5'-F1c-FL-F1-N5'-3' ... (c)
[wobei N5' eine Schmelzregion darstellt, die aus einer zu der Basensequenz N5c der ersten Matrizennukleinsäure komplementären Basensequenz besteht, FL eine Schleifenregion darstellt, die die Basensequenz F2 umfasst, und F1c und F1 Stammregionen darstellen, die zueinander komplementäre Basensequenzen umfassen und in der Lage sind, einen Doppelstrang auszubilden.]
und mit einer verlängerungshemmenden Modifikation an einem 3'-Ende
und der ersten Matrizennukleinsäure, das Synthetisieren eines komplementären Strangs des Fw-Adapter-Nukleotids, ausgehend von einem 3'-Ende der ersten Matrizennukleinsäure, und das Erhalten einer zweiten Matrizennukleinsäure beinhaltet, die die folgende Struktur (d) umfasst:
5'-B1c-BL-B1-N3c-N5c-F1c-FLc-F1-3' ... (d)
[wobei FLc eine Basensequenz darstellt, die komplementär zu FL des Fw-Adapter-Nukleotids ist.]
in dem eine Stamm-Schleifen-Struktur an ein 3'-Ende der ersten Matrizennukleinsäure hinzugefügt wird; und
Schritt C, der das Amplifizieren der Basensequenz Nc der zweiten Matrizennukleinsäure durch ein LAMP-Verfahren durch Verwenden eines inneren Fw-Primers, der die folgende Struktur (e) umfasst:
5'-F1c-F2-3' ... (e)
und eines inneren Bw-Primers, der die folgende Struktur (f) umfasst:
5'-B1c-B2-3' ... (f)
mit der zweiten Matrizennukleinsäure als eine Matrize beinhaltet.

2. Verfahren zum Amplifizieren von Nukleinsäuren nach Anspruch 1, wobei eine Länge der Basensequenz Nc 10 bis 100 Basen beträgt.

3. Verfahren zum Amplifizieren von Nukleinsäuren nach Anspruch 1 oder 2, wobei die Zielnukleinsäure miRNA ist.

4. Verfahren zum Amplifizieren von Nukleinsäuren nach einem der Ansprüche 1 bis 3, wobei Schritt A und Schritt B in demselben Reaktionssystem ablaufen.

5. Verfahren zum Amplifizieren von Nukleinsäuren nach einem der Ansprüche 1 bis 4, ferner umfassend: einen Wärmebehandlungsschritt des Erwärmens des Reaktionsprodukts von Schritt B auf 85 °C oder höher nach Schritt B und vor Schritt C.

6. Verfahren zum Amplifizieren von Nukleinsäuren nach einem der Ansprüche 1 bis 5, ferner umfassend: nach Schritt C einen Nachweisschritt des Nachweises der Basensequenz Nc unter Verwendung einer Sonde, die mit wenigstens einem Teil der Basensequenz Nc oder wenigstens einem Teil eines komplementären Strangs der Basensequenz Nc hybridisiert.

7. Verfahren zum Amplifizieren von Nukleinsäuren nach Anspruch 6, wobei in der Sonde eine Länge einer Basensequenz, die mit der Basensequenz N3c hybridisiert, 5 Basen oder weniger beträgt.

8. Verfahren zum Amplifizieren von Nukleinsäuren nach Anspruch 6, wobei eine Basensequenz in voller Länge der Sonde nicht vollständig in einer der Basensequenzen N3c und der Basensequenz N5c enthalten ist.

9. Verwendung eines Primer- und Adaptersets in dem Verfahren zum Amplifizieren von Nukleinsäuren nach einem der Ansprüche 1 bis 8, wobei das Primer- und Adapterset umfasst:
einen Bw-Adapterprimer, umfassend die folgende Struktur (a):
5'-B1c-BL-B1-N3c-3' ... (a)
[wobei N3c eine Glühregion darstellt, die aus einer Basensequenz besteht, die komplementär zu einer Basensequenz an einem 3'-Ende einer Zielregion einer Zielnukleinsäure ist, BL eine Schleifenregion darstellt, die die Basensequenz B2 umfasst, und B1c und B1 Stammregionen darstellen, die zueinander komplementäre Basensequenzen umfassen und in der Lage sind, einen Doppelstrang auszubilden.]
und ein Fw-Adapternukleotid, umfassend die folgenden Struktur:
5'-F1c-FL-F1-N5'-3' ... (c)
[wobei N5' eine Glühregion darstellt, die aus einer zu der Basensequenz N5c der ersten Matrizennukleinsäure komplementären Basensequenz besteht, FL eine Schleifenregion darstellt, die die Basensequenz F2 umfasst, und F1c und F1 Stammregionen darstellen, die zueinander komplementäre Basensequenzen umfassen und in der Lage sind, einen Doppelstrang auszubilden.]
und mit einer verlängerungshemmenden Modifikation an dem 3'-Ende.

10. Verwendung nach Anspruch 9, wobei der Primer- und Adaptersatz ferner umfasst:
Einen inneren Fw-Primer, umfassend die folgende Struktur (e):
5'-F1c-F2-3' ... (e)
und
einen inneren Bw-Primer, umfassend die folgende Struktur (f):
5'-B1c-B2-3' ... (f).

11. Verwendung eines Kits in dem Verfahren zum Amplifizieren von Nukleinsäuren nach einem der Ansprüche 1 bis 8, wobei das Kit umfasst:
(i) den Primer- und Adaptersatz nach Anspruch 9 oder 10; und
(ii) eine Sonde, die mit wenigstens einem Teil der Basensequenz Nc oder wenigstens einem Teil eines komplementären Strangs der Basensequenz Nc hybridisiert.

12. Verwendung nach Anspruch 11, wobei in der Probe eine Länge einer Basensequenz, die mit der Basensequenz N3c hybridisiert, 5 Basen oder weniger beträgt.

13. Verwendung nach Anspruch 11, wobei eine Basensequenz in voller Länge der Sonde nicht vollständig in einer der Basensequenz N3c und der Basensequenz N5c enthalten ist.

## Revendications

1. Procédé pour amplifier des acides nucléiques, comprenant :
une étape A incluant le recuit d'une amorce adaptatrice Bw comprenant la structure suivante (a)
5'-B1c-BL-B1-N3c-3 ... (a)
[ici, N3c représente une région de recuit composée d'une séquence de bases complémentaire à une séquence de bases sur un côté extrémité 3' d'une région cible d'un acide nucléique cible, BL représente une région de boucle comprenant la séquence de bases B2, et B1c et B1 représentent des régions de tige comprenant des séquences de bases mutuellement complémentaires et capables de former un double brin]
et la région cible, la synthétisation d'une séquence de bases complémentaire avec une séquence de bases sur un côté extrémité 5' de la région cible en commençant à partir d'une extrémité 3' de l'amorce adaptatrice Bw, et l'obtention d'un premier acide nucléique matrice comprenant la structure suivante (b) :
5' -B1c-BL-B1-N3c-N5c-3' ... (b)
[ici, N5c représente la séquence de bases complémentaire à la séquence de bases sur le côté extrémité 5' de la région cible, et une séquence de bases comprenant N3c, et N5c représente une séquence de bases Nc complémentaire à la région cible]
dans lequel une structure tige-boucle est ajoutée à une extrémité 5' d'un brin complémentaire de la région cible ;
l'étape B incluant le recuit d'un nucléotide adaptateur Fw comprenant la structure suivante (c)
5'-F1c-FL-F1-N5'-3' ... (c)
[ici, N5' représente une région de recuit composée d'une séquence de bases complémentaire à la séquence de bases N5c du premier acide nucléique matrice, FL représente une région de boucle comprenant la séquence de bases F2, et F1c et F1 représentent des régions de tige comprenant des séquences de bases mutuellement complémentaires et capables de former un double brin]
et ayant une modification inhibitrice d'extension à une extrémité 3'
et le premier acide nucléique matrice, la synthétisation d'un brin complémentaire du nucléotide adaptateur Fw en commençant à partir d'une extrémité 3' du premier acide nucléique matrice, et l'obtention d'un second acide nucléique matrice comprenant la structure suivante (d) :
5'-B1c-BL-B1-N3c-N5c-F1c-FLc-F1-3' ... (d)
[ici, FLc représente une séquence de bases complémentaire à FL du nucléotide adaptateur Fw]
dans lequel une structure tige-boucle est ajoutée à une extrémité 3' du premier acide nucléique matrice ; et
l'étape C incluant l'amplification de la séquence de bases Nc du second acide nucléique matrice par une méthode LAMP en utilisant une amorce intérieure Fw comprenant la structure suivante (e) :
5'-F1c-F2-3' ... (e)
et une amorce intérieure Bw comprenant la structure suivante (f) :
5'-B1c-B2-3' ... (f)
avec le second acide nucléique matrice en tant que matrice.

2. Procédé d'amplification d'acides nucléiques selon la revendication 1, dans lequel une longueur de la séquence de bases Nc est de 10 à 100 bases.

3. Procédé d'amplification d'acides nucléiques selon la revendication 1 ou 2, dans lequel l'acide nucléique cible est miARN.

4. Procédé d'amplification d'acides nucléiques selon l'une quelconque des revendications 1 à 3, dans lequel l'étape A et l'étape B se poursuivent dans le même système de réaction.

5. Procédé d'amplification d'acides nucléiques selon l'une quelconque des revendications 1 à 4, comprenant en outre : une étape de traitement par chaleur, du chauffage du produit de réaction de l'étape B jusqu'à 85 °C ou plus après l'étape B et avant l'étape C.

6. Procédé d'amplification d'acides nucléiques selon l'une quelconque des revendications 1 à 5, comprenant en outre : après l'étape C, une étape de détection, de la détection de la séquence de bases Nc en utilisant une sonde qui s'hybride avec au moins une partie de la séquence de bases Nc ou au moins une partie d'un brin complémentaire de la séquence de bases Nc.

7. Procédé d'amplification d'acides nucléiques selon la revendication 6, dans lequel, dans la sonde, une longueur d'une séquence de bases qui s'hybride avec la séquence de bases N3c est de 5 bases ou moins.

8. Procédé d'amplification d'acides nucléiques selon la revendication 6, dans lequel une séquence de bases de longueur complète de la sonde n'est pas contenue entièrement dans une de la séquence de bases N3c et de la séquence de bases N5c.

9. Utilisation d'un ensemble amorce et adaptateur dans le procédé d'amplification d'acides nucléiques selon l'une quelconque des revendications 1 à 8, dans lequel l'ensemble amorce et adaptateur comprend :
une amorce adaptatrice Bw comprenant la structure suivante (a) :
5'-B1c-BL-B1-N3c-3' ... (a)
[ici, N3c représente une région de recuit composée d'une séquence de bases complémentaire à une séquence de bases sur un côté extrémité 3' d'une région cible d'un acide nucléique cible, BL représente une région de boucle comprenant la séquence de bases B2, et B1c et B1 représentent des régions de tige comprenant des séquences de bases mutuellement complémentaires et capables de former un double brin]
et un nucléotide adaptateur Fw comprenant la structure suivante (c) :
5'-F1c-FL-F1-N5'-3' ... (c)
[ici, N5' représente une région de recuit composée d'une séquence de bases complémentaire à séquence de bases N5c du premier acide nucléique matrice, FL représente une région de boucle comprenant la séquence de bases F2, et F1c et F1 représentent des régions de tige comprenant des séquences de bases mutuellement complémentaires et capables de former un double brin]
et ayant une modification inhibitrice d'extension à l'extrémité 3'.

10. Utilisation selon la revendication 9, dans laquelle l'ensemble amorce et adaptateur comprend en outre :
une amorce intérieure Fw comprenant la structure suivante (e)
5' -F1c-F2-3' ... (e)
et
une amorce intérieure Bw comprenant la structure suivante (f)
5' -B1c-B2-3' ... (f) .

11. Utilisation d'un kit dans le procédé d'amplification d'acides nucléiques selon l'une quelconque des revendications 1 à 8, dans laquelle le kit comprend :
(i) l'ensemble amorce et adaptateur selon la revendication 9 ou 10 ; et
(ii) une sonde qui s'hybride avec au moins une partie de la séquence de bases Nc ou au moins une partie d'un brin complémentaire de la séquence de bases Nc.

12. Utilisation selon la revendication 11, dans laquelle, dans la sonde, une longueur d'une séquence de bases qui s'hybride avec la séquence de bases N3c est de 5 bases ou moins.

13. Utilisation selon la revendication 11, dans laquelle une séquence de bases de longueur complète de la sonde n'est pas contenue entièrement dans une de la séquence de bases N3c et de la séquence de bases N5c.
